(19) Europäisches Patentamt / European Patent Office / Office européen des brevets

(11) **EP 4 306 657 A1**

(12) **EUROPEAN PATENT APPLICATION**
published in accordance with Art. 153(4) EPC

(43) Date of publication:
**17.01.2024 Bulletin 2024/03**

(51) International Patent Classification (IPC):
***C12Q 1/6886*** (2018.01)   ***G01N 33/574*** (2006.01)

(21) Application number: **22767462.9**

(52) Cooperative Patent Classification (CPC):
**C12Q 1/6886; G01N 33/574**

(22) Date of filing: **08.03.2022**

(86) International application number:
**PCT/KR2022/003242**

(87) International publication number:
**WO 2022/191566 (15.09.2022 Gazette 2022/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.03.2021 KR 20210029877**

(71) Applicant: **Acurasysbio Co., Ltd.**
**Seoul 06229 (KR)**

(72) Inventors:
• **LEE, Hyung-Keun**
**Seongnam-si Gyeonggi-do 13530 (KR)**
• **LEE, Dong-Ki**
**Seongnam-si Gyeonggi-do 13556 (KR)**
• **KIM, So-Young**
**Suwon-si Gyeonggi-do 16357 (KR)**
• **JANG, Sung-Ill**
**Seoul 06217 (KR)**

(74) Representative: **Schnappauf, Georg**
**ZSP Patentanwälte PartG mbB**
**Hansastraße 32**
**80686 München (DE)**

Remarks:
The complete document including Reference Table(s) and the Sequence Listing(s) can be downloaded from the EPO website

(54) **COMPOSITION FOR DIAGNOSING PANCREATIC CANCER**

(57)    The present invention relates to a composition, a kit, and a method of providing information for diagnosis, which are capable of diagnosing pancreatic cancer and capable of diagnosing pancreatic cancer by distinguishing it from other pancreatic diseases or other cancer types.

[FIG. 1]

| Cutoff | Sensitivity% | 95% CI | Specificity% | 95% CI | Likelihood ratio |
|---|---|---|---|---|---|
| > 6.302 | 95.83 | 86.02% to 99.26% | 89.39 | 79.69% to 94.77% | 9.036 |
| > 6.306 | 93.75 | 83.16% to 97.85% | 89.39 | 79.69% to 94.77% | 8.839 |
| > 6.311 | 89.58 | 77.83% to 95.47% | 89.39 | 79.69% to 94.77% | 8.446 |
| > 6.338 | 89.58 | 77.83% to 95.47% | 90.91 | 81.55% to 95.77% | 9.854 |
| **> 6.382** | **89.58** | 77.83% to 95.47% | **92.42** | 83.46% to 96.72% | 11.83 |
| > 6.413 | 87.50 | 75.30% to 94.14% | 92.42 | 83.46% to 96.72% | 11.55 |
| > 6.429 | 87.50 | 75.30% to 94.14% | 93.94 | 85.43% to 97.62% | 14.44 |
| > 6.442 | 87.50 | 75.30% to 94.14% | 95.45 | 87.47% to 98.76% | 19.25 |

EP 4 306 657 A1

**Sequence Listing Free Text**

<110>   ACURASYSBIO Co., Ltd

<120>      Composition for diagnosing pancreatic cancer

<130>      OP22-002PCT

<150>      KR 10-2021-0029877

<151>      2021-03-08

<160>      5

<170>      KoPatentIn 3.0

<210>      1

<211>     506

<212>     PRT

<213>     Homo sapiens

<400>     1

Met Leu Lys Pro Leu Trp Lys Ala Ala Val Ala Pro Thr Trp Pro Cys

1                 5                 10                15

Ser Met Pro Pro Arg Arg Pro Trp Asp Arg Glu Ala Gly Thr Leu Gln

            20                25                30

Val Leu Gly Ala Leu Ala Val Leu Trp Leu Gly Ser Val Ala Leu Ile

      35                40                45

Cys Leu Leu Trp Gln Val Pro Arg Pro Pro Thr Trp Gly Gln Val Gln

      50                55                60

Pro Lys Asp Val Pro Arg Ser Trp Glu His Gly Ser Ser Pro Ala Trp

      65                70                75                80

Glu Pro Leu Glu Ala Glu Ala Arg Gln Gln Arg Asp Ser Cys Gln Leu

                85                90                95

Val Leu Val Glu Ser Ile Pro Gln Asp Leu Pro Ser Ala Ala Gly Ser

                100               105               110

Pro Ser Ala Gln Pro Leu Gly Gln Ala Trp Leu Gln Leu Leu Asp Thr

            115               120               125

Ala Gln Glu Ser Val His Val Ala Ser Tyr Tyr Trp Ser Leu Thr Gly

      130               135               140

Pro Asp Ile Gly Val Asn Asp Ser Ser Ser Gln Leu Gly Glu Ala Leu

145               150               155               160

Leu Gln Lys Leu Gln Gln Leu Leu Gly Arg Asn Ile Ser Leu Ala Val

165 170 175

Ala Thr Ser Ser Pro Thr Leu Ala Arg Thr Ser Thr Asp Leu Gln Val

180 185 190

Leu Ala Ala Arg Gly Ala His Val Arg Gln Val Pro Met Gly Arg Leu

195 200 205

Thr Arg Gly Val Leu His Ser Lys Phe Trp Val Val Asp Gly Arg His

210 215 220

Ile Tyr Met Gly Ser Ala Asn Met Asp Trp Arg Ser Leu Thr Gln Val

225 230 235 240

Lys Glu Leu Gly Ala Val Ile Tyr Asn Cys Ser His Leu Ala Gln Asp

245 250 255

Leu Glu Lys Thr Phe Gln Thr Tyr Trp Val Leu Gly Val Pro Lys Ala

260 265 270

Val Leu Pro Lys Thr Trp Pro Gln Asn Phe Ser Ser His Phe Asn Arg

275 280 285

Phe Gln Pro Phe His Gly Leu Phe Asp Gly Val Pro Thr Thr Ala Tyr

290 295 300

Phe Ser Ala Ser Pro Pro Ala Leu Cys Pro Gln Gly Arg Thr Arg Asp

305 310 315 320

Leu Glu Ala Leu Leu Ala Val Met Gly Ser Ala Gln Glu Phe Ile Tyr

325 330 335

Ala Ser Val Met Glu Tyr Phe Pro Thr Thr Arg Phe Ser His Pro Pro

340 345 350

Arg Tyr Trp Pro Val Leu Asp Asn Ala Leu Arg Ala Ala Ala Phe Gly

355 360 365

Lys Gly Val Arg Val Arg Leu Leu Val Gly Cys Gly Leu Asn Thr Asp

370 375 380

Pro Thr Met Phe Pro Tyr Leu Arg Ser Leu Gln Ala Leu Ser Asn Pro

385 390 395 400

Ala Ala Asn Val Ser Val Asp Val Lys Val Phe Ile Val Pro Val Gly

405 410 415

Asn His Ser Asn Ile Pro Phe Ser Arg Val Asn His Ser Lys Phe Met

420 425 430

Val Thr Glu Lys Ala Ala Tyr Ile Gly Thr Ser Asn Trp Ser Glu Asp

435 440 445

Tyr Phe Ser Ser Thr Ala Gly Val Gly Leu Val Val Thr Gln Ser Pro

450 455 460

Gly Ala Gln Pro Ala Gly Ala Thr Val Gln Glu Gln Leu Arg Gln Leu

465 470 475 480

Phe Glu Arg Asp Trp Ser Ser Arg Tyr Ala Val Gly Leu Asp Gly Gln

485 490 495

Ala Pro Gly Gln Asp Cys Val Trp Gln Gly

500 505

<210>    2

<211>    598

<212>    PRT

<213>    Homo sapiens

<400>    2

Met Pro Cys Ile Gln Ala Gln Tyr Gly Thr Pro Ala Pro Ser Pro Gly

```
        1              5                  10                  15

Pro Arg Asp His Leu Ala Ser Asp Pro Leu Thr Pro Glu Phe Ile Lys

               20                  25                  30

Pro Thr Met Asp Leu Ala Ser Pro Glu Ala Ala Pro Ala Ala Pro Thr

               35                  40                  45

Ala Leu Pro Ser Phe Ser Thr Phe Met Asp Gly Tyr Thr Gly Glu Phe

               50                  55                  60

Asp Thr Phe Leu Tyr Gln Leu Pro Gly Thr Val Gln Pro Cys Ser Ser

        65                  70                  75                  80

Ala Ser Ser Ser Ala Ser Ser Thr Ser Ser Ser Ser Ala Thr Ser Pro

                    85                  90                  95

Ala Ser Ala Ser Phe Lys Phe Glu Asp Phe Gln Val Tyr Gly Cys Tyr

                    100                 105                 110

Pro Gly Pro Leu Ser Gly Pro Val Asp Glu Ala Leu Ser Ser Ser Gly

                    115                 120                 125

Ser Asp Tyr Tyr Gly Ser Pro Cys Ser Ala Pro Ser Pro Ser Thr Pro

                    130                 135                 140

Ser Phe Gln Pro Pro Gln Leu Ser Pro Trp Asp Gly Ser Phe Gly His

        145                 150                 155                 160

Phe Ser Pro Ser Gln Thr Tyr Glu Gly Leu Arg Ala Trp Thr Glu Gln

                    165                 170                 175

Leu Pro Lys Ala Ser Gly Pro Pro Gln Pro Pro Ala Phe Phe Ser Phe

                    180                 185                 190

Ser Pro Pro Thr Gly Pro Ser Pro Ser Leu Ala Gln Ser Pro Leu Lys

                    195                 200                 205
```

Leu Phe Pro Ser Gln Ala Thr His Gln Leu Gly Glu Gly Glu Ser Tyr
210 215 220

Ser Met Pro Thr Ala Phe Pro Gly Leu Ala Pro Thr Ser Pro His Leu
225 230 235 240

Glu Gly Ser Gly Ile Leu Asp Thr Pro Val Thr Ser Thr Lys Ala Arg
245 250 255

Ser Gly Ala Pro Gly Gly Ser Glu Gly Arg Cys Ala Val Cys Gly Asp
260 265 270

Asn Ala Ser Cys Gln His Tyr Gly Val Arg Thr Cys Glu Gly Cys Lys
275 280 285

Gly Phe Phe Lys Arg Thr Val Gln Lys Asn Ala Lys Tyr Ile Cys Leu
290 295 300

Ala Asn Lys Asp Cys Pro Val Asp Lys Arg Arg Arg Asn Arg Cys Gln
305 310 315 320

Phe Cys Arg Phe Gln Lys Cys Leu Ala Val Gly Met Val Lys Glu Val
325 330 335

Val Arg Thr Asp Ser Leu Lys Gly Arg Arg Gly Arg Leu Pro Ser Lys
340 345 350

Pro Lys Gln Pro Pro Asp Ala Phe Pro Ala Asn Leu Leu Thr Ser Leu
355 360 365

Val Arg Ala His Leu Asp Ser Gly Pro Ser Thr Ala Lys Leu Asp Tyr
370 375 380

Ser Lys Phe Gln Glu Leu Val Leu Pro His Phe Gly Lys Glu Asp Ala
385 390 395 400

Gly Asp Val Gln Gln Phe Tyr Asp Leu Leu Ser Gly Ser Leu Glu Val

405 410 415

Ile Arg Lys Trp Ala Glu Lys Ile Pro Gly Phe Ala Glu Leu Ser Pro

420 425 430

Ala Asp Gln Asp Leu Leu Leu Glu Ser Ala Phe Leu Glu Leu Phe Ile

435 440 445

Leu Arg Leu Ala Tyr Arg Ser Lys Pro Gly Glu Gly Lys Leu Ile Phe

450 455 460

Cys Ser Gly Leu Val Leu His Arg Leu Gln Cys Ala Arg Gly Phe Gly

465 470 475 480

Asp Trp Ile Asp Ser Ile Leu Ala Phe Ser Arg Ser Leu His Ser Leu

485 490 495

Leu Val Asp Val Pro Ala Phe Ala Cys Leu Ser Ala Leu Val Leu Ile

500 505 510

Thr Asp Arg His Gly Leu Gln Glu Pro Arg Arg Val Glu Glu Leu Gln

515 520 525

Asn Arg Ile Ala Ser Cys Leu Lys Glu His Val Ala Ala Val Ala Gly

530 535 540

Glu Pro Gln Pro Ala Ser Cys Leu Ser Arg Leu Leu Gly Lys Leu Pro

545 550 555 560

Glu Leu Arg Thr Leu Cys Thr Gln Gly Leu Gln Arg Ile Phe Tyr Leu

565 570 575

Lys Leu Glu Asp Leu Val Pro Pro Pro Pro Ile Ile Asp Lys Ile Phe

580 585 590

Met Asp Thr Leu Pro Phe

595

<210> 3

<211> 231

<212> PRT

<213> Homo sapiens

<400> 3

Met Gln Asp Glu Glu Arg Tyr Met Thr Leu Asn Val Gln Ser Lys Lys
1               5                   10                  15

Arg Ser Ser Ala Gln Thr Ser Gln Leu Thr Phe Lys Asp Tyr Ser Val
                20                  25                  30

Thr Leu His Trp Tyr Lys Ile Leu Leu Gly Ile Ser Gly Thr Val Asn
            35                  40                  45

Gly Ile Leu Thr Leu Thr Leu Ile Ser Leu Ile Leu Leu Val Ser Gln
        50                  55                  60

Gly Val Leu Leu Lys Cys Gln Lys Gly Ser Cys Ser Asn Ala Thr Gln
    65                  70                  75                  80

Tyr Glu Asp Thr Gly Asp Leu Lys Val Asn Asn Gly Thr Arg Arg Asn
                85                  90                  95

Ile Ser Asn Lys Asp Leu Cys Ala Ser Arg Ser Ala Asp Gln Thr Val
                100                 105                 110

Leu Cys Gln Ser Glu Trp Leu Lys Tyr Gln Gly Lys Cys Tyr Trp Phe
            115                 120                 125

Ser Asn Glu Met Lys Ser Trp Ser Asp Ser Tyr Val Tyr Cys Leu Glu
        130                 135                 140

Arg Lys Ser His Leu Leu Ile Ile His Asp Gln Leu Glu Met Ala Phe

        145              150              155              160

Ile Gln Lys Asn Leu Arg Gln Leu Asn Tyr Val Trp Ile Gly Leu Asn

                 165              170              175

Phe Thr Ser Leu Lys Met Thr Trp Thr Trp Val Asp Gly Ser Pro Ile

                 180              185              190

Asp Ser Lys Ile Phe Phe Ile Lys Gly Pro Ala Lys Glu Asn Ser Cys

                195              200              205

Ala Ala Ile Lys Glu Ser Lys Ile Phe Ser Glu Thr Cys Ser Ser Val

            210              215              220

Phe Lys Trp Ile Cys Gln Tyr

225              230


<210> 4

<211> 119

<212> PRT

<213> Homo sapiens

<400> 4

Met Lys Ala Leu Ser Pro Val Arg Gly Cys Tyr Glu Ala Val Cys Cys

      1               5               10              15

Leu Ser Glu Arg Ser Leu Ala Ile Ala Arg Gly Arg Gly Lys Gly Pro

                 20              25              30

Ala Ala Glu Glu Pro Leu Ser Leu Leu Asp Asp Met Asn His Cys Tyr

                35              40              45

Ser Arg Leu Arg Glu Leu Val Pro Gly Val Pro Arg Gly Thr Gln Leu

        50               55              60

Ser Gln Val Glu Ile Leu Gln Arg Val Ile Asp Tyr Ile Leu Asp Leu

65               70               75               80

Gln Val Val Leu Ala Glu Pro Ala Pro Gly Pro Pro Asp Gly Pro His

85               90               95

Leu Pro Ile Gln Thr Ala Glu Leu Ala Pro Glu Leu Val Ile Ser Asn

100            105            110

Asp Lys Arg Ser Phe Cys His

115


<210>     5

<211>     459

<212>     PRT

<213>     Homo sapiens

<400>     5

Met Thr Ile Leu Gly Thr Thr Phe Gly Met Val Phe Ser Leu Leu Gln

1               5               10               15

Val Val Ser Gly Glu Ser Gly Tyr Ala Gln Asn Gly Asp Leu Glu Asp

20               25               30

Ala Glu Leu Asp Asp Tyr Ser Phe Ser Cys Tyr Ser Gln Leu Glu Val

35               40               45

Asn Gly Ser Gln His Ser Leu Thr Cys Ala Phe Glu Asp Pro Asp Val

50               55               60

Asn Ile Thr Asn Leu Glu Phe Glu Ile Cys Gly Ala Leu Val Glu Val

65               70               75               80

Lys Cys Leu Asn Phe Arg Lys Leu Gln Glu Ile Tyr Phe Ile Glu Thr

85              90             95

Lys Lys Phe Leu Leu Ile Gly Lys Ser Asn Ile Cys Val Lys Val Gly

100           105          110

Glu Lys Ser Leu Thr Cys Lys Lys Ile Asp Leu Thr Thr Ile Val Lys

115           120          125

Pro Glu Ala Pro Phe Asp Leu Ser Val Ile Tyr Arg Glu Gly Ala Asn

130           135          140

Asp Phe Val Val Thr Phe Asn Thr Ser His Leu Gln Lys Lys Tyr Val

145           150          155          160

Lys Val Leu Met His Asp Val Ala Tyr Arg Gln Glu Lys Asp Glu Asn

165           170          175

Lys Trp Thr His Val Asn Leu Ser Ser Thr Lys Leu Thr Leu Leu Gln

180           185          190

Arg Lys Leu Gln Pro Ala Ala Met Tyr Glu Ile Lys Val Arg Ser Ile

195           200          205

Pro Asp His Tyr Phe Lys Gly Phe Trp Ser Glu Trp Ser Pro Ser Tyr

210           215          220

Tyr Phe Arg Thr Pro Glu Ile Asn Asn Ser Ser Gly Glu Met Asp Pro

225           230          235          240

Ile Leu Leu Thr Ile Ser Ile Leu Ser Phe Phe Ser Val Ala Leu Leu

245           250          255

Val Ile Leu Ala Cys Val Leu Trp Lys Lys Arg Ile Lys Pro Ile Val

260           265          270

Trp Pro Ser Leu Pro Asp His Lys Lys Thr Leu Glu His Leu Cys Lys

275           280          285

Lys Pro Arg Lys Asn Leu Asn Val Ser Phe Asn Pro Glu Ser Phe Leu

290            295            300

Asp Cys Gln Ile His Arg Val Asp Asp Ile Gln Ala Arg Asp Glu Val

305            310            315            320

Glu Gly Phe Leu Gln Asp Thr Phe Pro Gln Gln Leu Glu Glu Ser Glu

325            330            335

Lys Gln Arg Leu Gly Gly Asp Val Gln Ser Pro Asn Cys Pro Ser Glu

340            345            350

Asp Val Val Ile Thr Pro Glu Ser Phe Gly Arg Asp Ser Ser Leu Thr

355            360            365

Cys Leu Ala Gly Asn Val Ser Ala Cys Asp Ala Pro Ile Leu Ser Ser

370            375            380

Ser Arg Ser Leu Asp Cys Arg Glu Ser Gly Lys Asn Gly Pro His Val

385            390            395            400

Tyr Gln Asp Leu Leu Leu Ser Leu Gly Thr Thr Asn Ser Thr Leu Pro

405            410            415

Pro Pro Phe Ser Leu Gln Ser Gly Ile Leu Thr Leu Asn Pro Val Ala

420            425            430

Gln Gly Gln Pro Ile Leu Thr Ser Leu Gly Ser Asn Gln Glu Glu Ala

435            440            445

Tyr Val Thr Met Ser Ser Phe Tyr Gln Asn Gln

450            455

<210> 6

<211> 21

<212>    DNA

<213>    Artificial Sequence

<220>

<223>    PLD4 forward primer

<400>    6

caaattctgg                                    gttgtggatg                          g

21


<210>    7

<211>    21

<212>    DNA

<213>    Artificial Sequence

<220>

<223>    PLD4 reverse primer

<400>    7

aggtggctgc                                    agttatagat                          g

21


<210>    8

<211>    18

<212>    DNA

<213>    Artificial Sequence

<220>

<223>    NR4A1 forward primer

<400>    8

gacaacgctt                                            catgccag

18

<210>      9

<211>     21

<212>    DNA

<213>    Artificial Sequence

<220>

<223>    NR4A1 reverse primer

<400>    9

ttgttagcca                        ggcagatgta                   c

21

<210>    10

<211>    21

<212>    DNA

<213>    Artificial Sequence

<220>

<223>    KLRF1 forward primer

<400>    10

gaaaaggagt                        tctgcccaaa                   c

21

<210>    11

<211>    22

<212>    DNA

<213>    Artificial Sequence

<220>

<223>    KLRF1 reverse primer

<400>    11

agaaaccaac                    aggatcaagg                    ag

22


<210>    12

<211>    21

<212>    DNA

<213>    Artificial Sequence

<220>

<223>    IL7R forward primer

<400>    12

gtagtcatca                    ctccagaaag                    c

21


<210>    13

<211>    21

<212>    DNA

<213>    Artificial Sequence

<220>

<223>    IL7R reverse primer

<400>    13

acctggaaga ggagagaata g

21

<210> 14

<211> 21

<212> DNA

<213> Artificial Sequence

<220>

<223> ID3 forward primer

<400> 14

gactacattc tcgacctgca g

21

<210> 15

<211> 21

<212> DNA

<213> Artificial Sequence

<220>

<223> ID3 reverse primer

<400> 15

tcgttggaga tgacaagttc c

21

<210> 16

<211> 23

<212> DNA

<213> Artificial Sequence

<220>

<223> PLD4-FAM probe

<400> 16

tctgacgcag          gtgaaggagc          ttg

23

<210> 17

<211> 25

<212> DNA

<213> Artificial Sequence

<220>

<223> IL7R-FAM probe

<400> 17

cagttggaag          tgaatggatc          gcagc

25

<210> 18

<211> 22

<212> DNA

<213> Artificial Sequence

<220>

<223> ID3-FAM probe

<400> 18

ctcggctgtc                    tggatgggaa                    gg

22

## Description

### Technical Field

The present invention relates to a composition and a kit for diagnosing pancreatic cancer, and a method of providing information for diagnosis of pancreatic cancer.

### Background Art

Studies on methods for treating and diagnosing cancer among major diseases in modern people have been relatively actively conducted, focusing on lung cancer, liver cancer, gastric cancer, etc., which occur at high incidence. However, studies on esophageal cancer, colorectal cancer, pancreatic cancer, etc., which occur at low incidence, are relatively insufficient.

In particular, pancreatic cancer does not show recognizable symptoms in its early stages, and usually shows symptoms such as pain and weight loss after systemic metastasis has already occurred, indicating that the cure rate of pancreatic cancer is relatively low. Thus, regular testing for pancreatic cancer is very important. Most of the clinical symptoms of pancreatic cancer gradually appear, and frail health, loss of appetite, and weight loss are the most common symptoms. Pancreatic cancer is a fatal cancer with a 5-year survival rate of 1 to 4% and a median survival period of 5 months, and has the poorest prognosis among human cancers. In addition, since 80 to 90% of pancreatic cancer patients are diagnosed in a state in which curative resection making a cure possible is not possible, the prognosis thereof is poor. Also, the treatment of pancreatic cancer mainly depends on chemotherapy. Thus, there is a more urgent need to develop an early diagnosis method for pancreatic cancer than any other human cancer.

Several anticancer drugs, including 5-fluorouracil, gemcitabine, and Tarceva, which are known to be effective for pancreatic cancer until now, have extremely low therapeutic effects, and the response rate to chemotherapy using these drugs is only 15%. This fact suggests that the development of a more effective early diagnosis method and treatment method for pancreatic cancer is urgently required in order to improve the prognosis of pancreatic cancer patients. Appropriate diagnosis and treatment of precursor lesions of pancreatic cancer, which are at a stage prior to progression to fatal pancreatic cancer, is very important to improve pancreatic cancer treatment results.

For the diagnosis of pancreatic cancer or precursor lesions of pancreatic cancer, blood test (CA19-9), gastro-duodenography with X-ray, cholangiography through the skin and liver, and retrograde endoscopic cholangiography are used. Although disease lesions have been detected by these methods, ultrasonography and computed tomography have recently been most commonly used. Relatively accurate test results may be obtained by performing a more precise biopsy. However, this diagnostic method has low accuracy, or subjects are unwilling to undergo the diagnostic method because the diagnostic method is inconvenient and painful. Therefore, there has been a demand for the development of a test method capable of diagnosing pancreatic cancer or precursor lesions of pancreatic cancer conveniently and rapidly.

Korean Patent No. 10-0819122 and Korean Patent Application Publication No. 2012-0082372 disclose technologies using various pancreatic cancer markers, including matrilin, transthyretin, stratifin, and the like. However, diagnostic efficiency and accuracy significantly differ between markers. Therefore, there is a need to discover more effective markers and develop a diagnostic method using the same.

## DISCLOSURE

### Technical Problem

An object of the present invention is to provide a composition for diagnosing pancreatic cancer.

Another object of the present invention is to provide a kit for diagnosing pancreatic cancer.

Still another object of the present invention is to provide a method of providing information for diagnosis of pancreatic cancer.

However, objects to be achieved by the present invention are not limited to the above-mentioned objects, and other objects not mentioned herein will be clearly understood by those skilled in the art from the following description.

### Technical Solution

One embodiment of the present invention is directed to a biomarker for diagnosing cancer comprising either at least one protein selected from the group consisting of PLD4 (phospholipase D family member 4), NR4A1 (nuclear receptor subfamily 4 group A member 1), KLRF1 (killer cell lectin like receptor F1), and ID3 (inhibitor of DNA binding 3, HLH protein), or a gene encoding the at least one protein.

In the present invention, the "PLD4 (phospholipase D family member 4)" is 5'-3' exonuclease PLD4, which degrades single-stranded DNA (ssDNA). The PLD4 functions to regulate an inflammatory cytokine response by reducing the concentration of ssDNA that stimulates TLR9 through nucleic acid degradation, and is known to be involved in phagocytosis of activated microglia. The PLD4 protein may consist of the amino acid sequence represented by SEQ ID NO: 1, without being limited thereto.

In the present invention, the "NR4A1 (nuclear receptor subfamily 4 group A member 1)" is also known as NGFIB (nerve growth factor IB) or Nur77, belongs to the family of intracellular transcription factors, and is known to be involved in cell cycle regulation, inflammation, or apoptosis. The NR4A1 may consist of the amino acid sequence represented by SEQ ID NO: 2, without being limited thereto.

In the present invention, the "KLRF1 (killer cell lectin like receptor F1)" is expressed in natural killer cells and is known to be involved in cytotoxicity and cytokine secretion. The KLRF1 may consist of the amino acid sequence represented by SEQ ID NO: 3, without being limited thereto.

In the present invention, the "ID3 (inhibitor of DNA binding 3, HLH protein)" is a helix-loop-helix (HLH) protein that forms a heterodimer with other HLH proteins. The ID3 protein lacks a DNA binding domain and thus inhibits the binding of other HLH proteins to DNA. The ID3 may consist of the amino acid sequence represented by SEQ ID NO: 4, without being limited thereto.

In the present invention, the biomarker may further comprise IL7R (interleukin 7 receptor) protein or a gene encoding the same.

In the present invention, the "IL7R (interleukin 7 receptor)" is a protein found on the cell surface, is made up of two different protein chains that form a heterodimer, and is composed of two subunits, CD127 and CD132. The IL7R is observed in various cells, including various naive and memory T cells, and is known to play an important role in the development of immune cells. The IL7R may consist of the amino acid sequence represented by SEQ ID NO: 5, without being limited thereto.

In one example of the present invention, the biomarker may comprise PLD4 and ID3, without being limited thereto.

In another example of the present invention, the biomarker may comprise PLD4, ID3 and IL7R, without being limited thereto.

In still another example of the present invention, the biomarker may comprise PLD4, ID3, NR4A1 and KLRF1, without being limited thereto.

In yet another example of the present invention, the biomarker may comprise PLD4, ID3, NR4A1, KLRF1 and IL7R, without being limited thereto.

In the present invention, the expression level of the biomarker, etc. may be measured from a biological sample isolated from a subject of interest, preferably a liquid biopsy, for example, blood, serum or plasma, or cells or exosomes isolated from the liquid biopsy.

In the present invention, the term "subject of interest" refers to individuals that have cancer or are highly likely to have cancer, wherein the individuals may be mammals, including humans. For example, the subject of interest may be selected from the group consisting of humans, rats, mice, guinea pigs, hamsters, rabbits, monkeys, dogs, cats, cows, horses, pigs, sheep, and goats, and is preferably a human, without being limited thereto.

In the present invention, when the expression level of the biomarker according to the present invention in a liquid biopsy isolated from a subject of interest or in cells or exosomes isolated from the liquid biopsy is measured, there is no need for an invasive process such as subjecting the patient to laparotomy and isolating tissue cells from tissue (e.g., pancreatic tissue), it takes less than about 5 minutes to obtain the biopsy, and it takes about less than 2 hours to measure the expression levels of the various disease biomarkers according to the present invention from the biopsy, suggesting that whether the disease has occurred or the likelihood of occurrence of the disease may be determined very rapidly and conveniently.

In the present invention, the term "cancer" refers to or describes the physiological condition typically characterized by unregulated cell growth in a mammal. The cancer may be thyroid cancer, parathyroid cancer, gastric cancer, ovarian cancer, colorectal cancer, pancreatic cancer, liver cancer, breast cancer, cervical cancer, lung cancer, non-small cell lung cancer, prostate cancer, gallbladder cancer, bile duct cancer, non-Hodgkin's lymphoma, Hodgkin's lymphoma, hematological cancer, bladder cancer, kidney cancer, melanoma, colon cancer, bone cancer, skin cancer, head cancer, uterine cancer, rectal cancer, brain tumor, perianal cancer, fallopian tube carcinoma, endometrial carcinoma, vaginal cancer, vulvar carcinoma, esophageal cancer, small intestine cancer, endocrine adenocarcinoma, adrenal cancer, soft tissue sarcoma, urethral cancer, penile cancer, cancer of ureter, renal cell carcinoma, renal pelvic carcinoma, central nervous system (CNS) tumor, primary CNS lymphoma, spinal cord tumor, brainstem glioma, or pituitary adenoma. Preferably, the cancer may be pancreatic cancer, but is not limited thereto and may be any type of cancer whose progression (such as tumor differentiation and/or proliferation) is dependent on the cancer cells or cancer stem cells described in the present invention.

In the present invention, the term "pancreatic cancer" refers to cancer originating from pancreatic cells. There are several types of pancreatic cancer, but pancreatic ductal adenocarcinomas originating from pancreatic ductal cells account for about 90%, and thus pancreatic cancer generally refers to pancreatic ductal adenocarcinoma. In addition, there are cystic cancer (cystadenocarcinoma), endocrine tumors, and the like. About 5% to 10% of pancreatic cancer patients have a genetic predisposition, and about 7.8% of pancreatic cancer patients have a family history of pancreatic cancer, which is higher than the incidence of pancreatic cancer in the general population (0.6%). Pancreatic cancer is a cancer with a very poor prognosis, with a 5-year survival rate of 5% or less. This is because most cases are diagnosed after the cancer has progressed, and thus cases in which surgical resection is possible at the time of diagnosis are less than 20%, and even if the tumor has been completely resected when viewed visually, there is little improvement in survival rate due to micrometastasis, and the responsiveness of the cancer to chemotherapy and radiotherapy is low. Therefore, the most important method to improve the survival rate is early detection and surgical removal of the cancer when there are no symptoms or nonspecific symptoms.

In the present invention, the term "diagnosis" means identifying the presence or characteristics of a pathological condition. For the purposes of the present invention, the term "diagnosis" may mean predicting the likelihood of occurrence, growth, progression, or metastasis of cancer, or may mean distinguishing cancer from other diseases, for example, a pancreatic disease, in particular, pancreatitis (including both acute and chronic), pancreatic benign tumors (lipoma or intraductal papillary mucinous neoplasm (IPMN), etc.), or may mean distinguishing between cancer types, particularly, distinguishing pancreatic cancer from other cancer types.

In the present invention, the term "pancreatitis" refers to diseases caused by inflammation of the pancreas, and includes acute pancreatitis and chronic pancreatitis. Pancreatic juice contains digestive enzymes such as amylase (which hydrolyzes carbohydrates), trypsin (which hydrolyzes proteins), and lipase (which hydrolyzes fats). Pancreatitis is not only caused by autolysis of the pancreas by the enzymes because pancreatic juice does not flow smoothly due to alcohol abuse, gallstones, etc., but also caused by various factors such as metabolic disorders, drugs, and abdominal damage. Pancreatitis is an inflammatory disease of the pancreas that causes pancreatic acinar cell damage, extensive interstitial edema, hemorrhage, and migration of neutrophilic granulocytes to the site of injury. Pancreatitis can be broadly divided into two types: mild type pancreatitis in which interstitial edema and peripancreatic fat necrosis are found; and severe type pancreatitis accompanied by extensive peri pancreatic and intrapancreatic fat necrosis, pancreatic parenchymal necrosis, and hemorrhage.

Another embodiment of the present invention is directed to a composition for diagnosing cancer containing, as an active ingredient, an agent for measuring the expression level of at least one protein selected from the group consisting of PLD4, NR4A1, KLRF1, and ID3, or a gene encoding the at least one protein.

In the present invention, the composition for diagnosing may further contain an agent for measuring the expression level of IL7R protein or a gene encoding the same.

In one example of the present invention, the composition for diagnosing may contain, as an active ingredient, an agent for measuring the expression levels of PLD4 and ID3 proteins or genes encoding the same, without being limited thereto.

In another example of the present invention, the composition for diagnosing may contain, as an active ingredient, an agent for measuring the expression levels of PLD4, ID3 and IL7R proteins or genes encoding the same, without being limited thereto.

In still another example of the present invention, the composition for diagnosing may contain, as an active ingredient, an agent for measuring the expression levels of PLD4, ID3, NR4A1 and KLRF1 proteins or genes encoding the same, without being limited thereto.

In yet another example of the present invention, the composition for diagnosing may contain, as an active ingredient, an agent for measuring the expression levels of PLD4, ID3, NR4A1, KLRF1 and IL7R proteins or genes encoding the same, without being limited thereto.

In the present invention, the agent for measuring the expression level of the protein may comprise at least one from the group consisting of an antibody, an oligopeptide, a ligand, a peptide nucleic acid (PNA), and an aptamer, which bind specifically to the protein, without being limited thereto.

In the present invention, the "antibody" refers to a substance that specifically binds to an antigen and causes an antigen-antibody reaction. For the purposes of the present invention, the antibody refers to an antibody that specifically binds to the protein. Examples of the antibody of the present invention include all of polyclonal antibodies, monoclonal antibodies, and recombinant antibodies. This antibody may be readily produced using techniques well known in the art. For example, a polyclonal antibody may be produced by a method well known in the art, which includes a process of obtaining a serum containing the antibody by injecting the antigen of the protein into an animal and collecting blood from the animal. This polyclonal antibody may be produced from any animal such as goat, rabbit, sheep, monkey, horse, pig, cow, dog, or the like. In addition, a monoclonal antibody may be produced using the hybridoma method well known in the art (see Kohler and Milstein (1976) European Journal of Immunology 6:511-519), or the phage antibody library technology (see Clackson et al, Nature, 352:624-628, 1991; Marks et al, J. Mol. Biol., 222:58, 1-597, 1991). The antibody produced by the above method may be isolated and purified using a method such as gel electrophoresis, dialysis, salt precipitation, ion exchange chromatography, or affinity chromatography. In addition, examples of the antibody of the present invention include not only a complete form having two full-length light chains and two full-length heavy chains, but also functional fragments of an antibody molecule. The phrase "functional fragment of an antibody molecule" refers to a fragment having at least an antigen-binding function, and examples of the fragment include Fab, F(ab'), F(ab')2, Fv, and the like.

In the present invention, the "oligopeptide" is a peptide composed of 2 to 20 amino acids and examples thereof include, but are not limited to, a dipeptide, tripeptide, tetrapeptide, and pentapeptide.

In the present invention, the term "PNA (Peptide Nucleic Acid)" refers to an artificially synthesized DNA or RNA-like polymer, which was first introduced by the Professors Nielsen, Egholm, Berg and Buchardt at University of Copenhagen, Denmark in 1991. DNA has a phosphate-ribose sugar backbone, but PNA has repeated N-(2-aminoethyl)-glycine backbones linked via peptide bonds, and thus has a significantly increased binding affinity for DNA or RNA and significantly increased stability. Thus, the PNA is used for molecular biology, diagnostic assays and antisense therapies. The PNA is disclosed in detail in the literature [Nielsen P E, Egholm M, Berg R H, Buchardt O (December 1991). "Sequence-selective recognition of DNA by strand displacement with a thymine-substituted polyamide". Science 254(5037): 1497-1500].

In the present invention, the term "aptamer" refers to an oligonucleotide or a peptide molecule, and general contents regarding the aptamer are disclosed in detail in the literature [Bock L C et al., Nature 355(6360):5646(1992); Hoppe-Seyler F, Butz K "Peptide aptamers: powerful new tools for molecular medicine". J Mol Med. 78(8):42630(2000); Cohen B A, Colas P, Brent R. "An artificial cell-cycle inhibitor isolated from a combinatorial library". Proc Natl Acad Sci USA. 95(24): 142727(1998)].

In the present invention, the agent for measuring the expression level of the gene encoding the protein may comprise at least one selected from the group consisting of a primer, a probe, and an antisense nucleotide, which bind specifically to the gene encoding the protein, without being limited thereto.

In the present invention, the term "primer" refers to a fragment that recognizes a target gene sequence. The primer comprises a pair of forward and reverse primers, but is preferably a pair of primers providing analysis results with specificity and sensitivity. When the nucleic acid sequence of the primer is a sequence inconsistent with the non-target sequence present in the sample, and thus is a primer that amplifies only the target gene sequence containing the complementary primer binding site without inducing nonspecific amplification, high specificity may be imparted.

In the present invention, the term "probe" refers to a substance capable of binding specifically to a target substance to be detected in a sample, and refers to a substance capable of specifically detecting the presence of the target substance in the sample through the binding. The type of probe is not particularly limited so long as it is commonly used in the art. Preferably, the probe may be PNA (peptide nucleic acid), LNA (locked nucleic acid), a peptide, a polypeptide, a protein, RNA or DNA. Most preferably, the probe is PNA. More specifically, the probe is a biomolecule derived from an organism or an analogue thereof, or is produced *in vitro.* Examples of the probe include an enzyme, a protein, an antibody, a microorganism, an animal and/or plant cell and organ, a neuron, DNA, and RNA. Examples of the DNA include cDNA, genomic DNA, and an oligonucleotide, examples of the RNA include genomic RNA, mRNA and an oligonucleotide, and examples of the protein include antibodies, antigens, enzymes, peptides, and the like.

In the present invention, the term "LNA (locked nucleic acid)" refers to a nucleic acid analogue containing a 2'-O or 4'-C methylene bridge [J Weiler, J Hunziker and J Hall Gene Therapy (2006) 13, 496.502]. LNA nucleosides comprise the common bases of DNA and RNA, and can form base pairs according to the Watson-Crick base-pair rule. However, LNA fails to form an ideal shape in the Watson-Crick bond due to "locking" of the molecule attributable to the methylene bridge. When LNA is incorporated in a DNA or RNA oligonucleotide, it can more rapidly pair with a complementary nucleotide chain, thus increasing the stability of the double strand.

In the present invention, the term "antisense" means an oligomer that has a nucleotide sequence and a backbone between subunits, wherein an antisense oligomer is hybridized with the target sequence in the RNA by Watson-Crick base pairing to typically allow the formation of the mRNA and RNA:oligomer heterodimers with the mRNA in the target sequence. The oligomer may have an accurate or approximate sequence complementarity to the target sequence.

Information on the PLD4, NR4A1, KLRF1, ID3 and IL7R proteins or the genes encoding these proteins according to the present invention is known. Thus, based on this information, any person skilled in the art will be able to easily design primers, probes or antisense nucleotides that bind specifically to the genes encoding the proteins.

In the present invention, the expression levels of the PLD4, NR4A1, KLRF1, ID3 and IL7R proteins or the genes encoding the same may be measured from a biological sample isolated from a subject of interest, preferably a liquid biopsy, for example, blood, serum or plasma, or cells or exosomes isolated from the liquid biopsy.

In the present invention, the cancer may be thyroid cancer, parathyroid cancer, gastric cancer, ovarian cancer, colorectal cancer, pancreatic cancer, liver cancer, breast cancer, cervical cancer, lung cancer, non-small cell lung cancer, prostate cancer, gallbladder cancer, bile duct cancer, non-Hodgkin's lymphoma, Hodgkin's lymphoma, hematological cancer, bladder cancer, kidney cancer, melanoma, colon cancer, bone cancer, skin cancer, head cancer, uterine cancer, rectal cancer, brain tumor, perianal cancer, fallopian tube carcinoma, endometrial carcinoma, vaginal cancer, vulvar carcinoma, esophageal cancer, small intestine cancer, endocrine adenocarcinoma, adrenal cancer, soft tissue sarcoma, urethral cancer, penile cancer, cancer of ureter, renal cell carcinoma, renal pelvic carcinoma, central nervous system (CNS) tumor, primary CNS lymphoma, spinal cord tumor, brainstem glioma, or pituitary adenoma. Preferably, the cancer may be pancreatic cancer, but is not limited thereto and may be any type of cancer whose progression (such as tumor differentiation and/or proliferation) is dependent on the cancer cells or cancer stem cells described in the present invention.

In the present invention, the composition for diagnosing may be used to predict the likelihood of occurrence, growth, progression, or metastasis of cancer, or may be used to distinguish cancer from other diseases, for example, a pancreatic disease, in particular, pancreatitis (including both acute and chronic), pancreatic benign tumors (lipoma or intraductal papillary mucinous neoplasm (IPMN), etc.), or may be used to diagnose pancreatic cancer by distinguishing between cancer types, particularly, distinguishing pancreatic cancer from other cancer types.

Still another embodiment of the present invention is directed to a kit for diagnosing cancer comprising the composition for diagnosing of the present invention.

In the present invention, the term "kit" refers to a tool in which a probe or antibody that binds specifically to a biomarker component is labeled with a detectable substance so that the expression level of the biomarker may be assessed. The kit may include not only a direct label that directly labels a substance capable of being detected with respect to a probe or antibody by a reaction with a substrate, but also an indirect label to which a marker that develops color by a reaction with another reagent, which is directly-labeled, is conjugated. The kit may comprise a chromogenic substrate solution to induce a chromogenic reaction with the label, a washing liquid, and other solutions, and may be prepared to comprise reagent components used. In the present invention, the kit may be a kit comprising essential components necessary for performing RT-PCR, and may comprise, in addition to each primer pair specific for the marker gene, a test tube, a reaction buffer, deoxynucleotides (dNTPs), Taq-polymerase, reverse transcriptase, DNase and RNase inhibitors, sterile water, and the like. The kit may also be a kit for detecting genes for cancer diagnosis comprising essential components necessary for performing DNA chip assay. The DNA chip kit may comprise a substrate to which a cDNA corresponding to a gene or a fragment thereof is attached as a probe, wherein the substrate may comprise a cDNA corresponding to a quantitative control gene or a fragment thereof. The kit of the present invention is not limited thereto and may be any type of kit known in the art.

In the present invention, the kit may be an RT-PCR kit, a DNA chip kit, an ELISA kit, a protein chip kit, a rapid kit, or a multiple reaction monitoring (MRM) kit.

The kit of the present invention may further include a composition, solution or device consisting of one or more types of different components, suitable for an analysis method. For example, the kit of the present invention may further include essential elements required for a reverse transcription polymerase chain reaction (RT-PCR). A kit for RT-PCR includes a primer pair specific for a marker protein-coding gene. The primers are nucleotides having a sequence specific for the nucleic acid sequence of the gene, having a length of approximately 7 bp to 50 bp, and more preferably, approximately 10 bp to 30 bp. In addition, the primers may include a primer specific for the nucleic acid sequence of a control gene. Alternatively, the kit for RT-PCR may include a test tube or another suitable container, a reaction buffer solution (pH and magnesium concentrations vary), deoxynucleotides (dNTPs), enzymes such as Taq-polymerase and reverse transcriptase, DNase, RNase inhibitor DEPC-water, and distilled water.

In addition, the kit of the present invention may include essential elements required for a DNA chip. The DNA chip kit may include a substrate to which cDNA or an oligonucleotide, which corresponds to a gene or a fragment thereof, is attached, and a reagent, agent and enzymes for preparing a fluorescence-labeled probe. In addition, the substrate may include cDNA or an oligonucleotide, which corresponds to a control gene or a fragment thereof, is attached.

In addition, the kit for diagnosing cancer according to the present invention may comprise essential components required for performing ELISA. The ELISA kit comprises an antibody specific to the protein. The antibody is a monoclonal antibody, a polyclonal antibody or a recombinant antibody, which has a high specificity and affinity for the marker protein and shows little or no cross-reactivity with other proteins. Also, the ELISA kit may comprise an antibody specific to a control protein. In addition, the ELISA kit may comprise reagents capable of detecting bound antibodies, for example labelled secondary antibodies, chromophores, enzymes (e.g., conjugated with antibodies) and the substrates thereof or other substances which are capable of binding to antibodies.

As a support for antigen-antibody interactions in the kit, a nitrocellulose membrane, a PVDF membrane, well plate synthesized of a polyvinyl resin or a polystyrene resin, or a slide glass formed of glass may be used, but the present invention is not limited thereto.

In addition, in the kit of the present invention, a label for a secondary antibody is preferably a conventional chromophore that develops color, and preferably, labels such as fluorescein or a dye such as poly L-lysine-fluorescein isothiocyanate (FITC), or rhodamine-B-isothiocyanate (RITC), horseradish peroxidase (HRP), alkaline phosphatase, colloidal gold, may be used, but the present invention is not limited thereto.

In addition, a chromogenic substrate for inducing color development in the kit of the present invention is preferably used according to a label that develops color, and may be 3,3',5,5'-tetramethyl benzidine (TMB), 2,2'-azino-bis(3-ethylbenzothiazolin-6-sulfonic acid) (ABTS) or o-phenylenediamine (OPD). Here, the chromogenic substrate is more preferably provided in a state of being dissolved in a buffer solution (0.1 M NaAc, pH 5.5). A chromogenic substrate such as TMB may be decomposed by HRP used as a label of a secondary antibody conjugate to generate a chromogenic deposit, and by visually observing the degree of deposition of the chromogenic deposit, the presence of the marker proteins is detected.

In the present invention, the washing solution preferably contains a phosphate buffer solution, NaCl, and Tween 20, and is more preferably a buffer solution (PBST) composed of a 0.02 M phosphate buffer solution, 0.13 M NaCl, and 0.05% Tween 20. After the secondary antibody is allowed to react with the antigen-antibody conjugate after the antigen-antibody binding reaction, a proper amount of the washing solution is added to the support to wash the support 3 to 6 times. A sulfuric acid ($H_2SO_4$) solution may be preferably used as the reaction stop solution.

Yet another embodiment of the present invention is directed to a method of providing information for diagnosis of cancer, the method comprising a step of measuring the expression level of at least one protein selected from the group consisting of PLD4, NR4A1, KLRF1 and ID3, or a gene encoding the at least one protein, in a biological sample isolated from a subject of interest.

In the present invention, the term "subject of interest" refers to individuals that have cancer or are highly likely to have cancer, wherein the individuals may be mammals, including humans. For example, the subject of interest may be selected from the group consisting of humans, rats, mice, guinea pigs, hamsters, rabbits, monkeys, dogs, cats, cows, horses, pigs, sheep, and goats, and is preferably a human, without being limited thereto.

In the present invention, the term "biological sample" refers to any substance, biological body fluid, tissue or cells obtained from or derived from the subject. The biological sample may be at least one selected from the group consisting of whole blood, leukocytes, peripheral blood mononuclear cells, buffy coat, plasma, serum, sputum, tears, mucus, nasal washes, nasal aspirate, breath, urine, semen, saliva, peritoneal washings, ascites, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extracts, and cerebrospinal fluid. Preferably, the biological sample may be cells or exosomes isolated from a liquid biopsy such as blood, serum or plasma.

In the present invention, the method of providing information may further comprise a step of measuring the expression level of IL7R protein or a gene encoding the same in the biological sample isolated from the subject of interest.

In one example of the present invention, the method of providing information may comprise a step of measuring the expression levels of PLD4 and ID3 proteins or genes encoding the same in the biological sample isolated from the subject of interest, without being limited thereto.

In another example of the present invention, the method of providing information may comprise a step of measuring the expression levels of PLD4, ID3 and IL7R proteins or genes encoding the same in the biological sample isolated from the subject of interest, without being limited thereto.

In still another example of the present invention, the method of providing information may comprise a step of measuring the expression levels of PLD4, ID3, NR4A1 and KLRF1 proteins or genes encoding the same in the biological sample isolated from the subject of interest, without being limited thereto.

In yet another example of the present invention, the method of providing information may comprise a step of measuring the expression levels of PLD4, ID3, NR4A1, KLRF1 and IL7R proteins or genes encoding the same in the biological sample isolated from the subject of interest, without being limited thereto.

In the present invention, the agent for measuring the expression level of the protein may comprise at least one from the group consisting of an antibody, an oligopeptide, a ligand, a peptide nucleic acid (PNA), and an aptamer, which bind specifically to the protein.

In the present invention, measurement of the expression level of the protein may be performed by protein chip assay, immunoassay, ligand binding assay, MALDI-TOF (Matrix Assisted Laser Desorption/Ionization Time of Flight) mass spectrometry, SELDI-TOF (Surface Enhanced Laser Desorption/Ionization Time of Flight) mass spectrometry, radioimmunoassay, radial immunodiffusion, Ouchterlony immunodiffusion, Rocket immunoelectrophoresis, immunohistostaining, complement fixation test, 2-D electrophoresis, liquid chromatography-mass spectrometry (LC-MS), liquid chromatography-mass spectrometry/mass spectrometry (LC-MS/MS), Western blotting, or enzyme-linked immunosorbent assay (ELISA).

In the present invention, measurement of the expression level of the protein may be performed by a multiple reaction monitoring (MRM) method.

In the present invention, in the multiple reaction monitoring method, either a synthetic peptide obtained by substituting a specific amino acid of a target peptide with an isotope or *E. coli* beta-galactosidase may be used as an internal standard.

In the present invention, the agent for measuring the expression level of the gene encoding the protein may comprise at least one selected from the group consisting of a primer, a probe, and an antisense nucleotide, which bind specifically to the gene encoding the protein.

In the present invention, measurement of the expression level of the gene encoding the protein may be performed by reverse transcription polymerase reaction (RT-PCR), competitive RT-PCR, real-time RT-PCR, RNase protection assay (RPA), Northern blotting, or DNA chip assay.

In the method of providing information according to the present invention, details regarding the antibody, oligopeptide, ligand, peptide nucleic acid (PNA), aptamer, etc., and details regarding the primer, probe, etc. overlap with those described above, and thus the detailed description thereof will be omitted below to avoid undue complexity of the specification.

In the present invention, when the expression level of at least one protein selected from the group consisting of PLD4, NR4A1 and KLRF1, or the gene encoding the at least one protein, measured in the biological sample isolated from the subject of interest, is lower than a control, it may be predicted that cancer has occurred or is highly likely to occur cancer in the subject of interest.

In the present invention, when the expression level of at least one protein selected from among ID3 and IL7R, or the gene encoding the at least one protein, measured in the biological sample isolated from the subject of interest, is higher than a control, it may be predicted that cancer has occurred or is highly likely to occur cancer in the subject of interest.

In the present invention, the term "control" may be, but is not limited to, the expression level of the corresponding biomarker protein or the gene encoding the protein in a healthy normal control group, or the average or median value of the expression level of the corresponding marker protein or the gene encoding the same in biological samples derived from patients with pancreatic disease, or the average value or median value of the expression level of the corresponding marker protein or the gene encoding the same in biological samples derived from cancer patients, preferably from patients with cancer types other than pancreatic cancer.

In the present invention, the method of providing information may further comprise a step of substituting, into Equation 1 below, the expression levels of PLD4, NR4A1, KLRF1, ID3 and IL7R proteins or the genes encoding the proteins, measured in the biological sample isolated from the subject of interest:

[Equation 1]

$A = a1 - b1 \times (PLD4) - c1 \times (NR4A1) - d1 \times (KLRF1) + e1 \times (IL7R) + f1 \times (ID3)$

wherein

al is a rational number of 1.5 to 2.0, preferably 1.75 to 2.0;
b1 is a rational number of 0.1 to 0.3, preferably 0.15 to 0.25;
c1 is a rational number of 0.01 to 0.03, preferably 0.02 to 0.03;
d1 is a rational number of 0.005 to 0.02, preferably 0.01 to 0.02;
e1 is a rational number of 0.0007 to 0.0015, preferably 0.0009 to 0.0015;
f1 is a rational number of 0.05 to 0.15, preferably 0.05 to 0.10; and
PLD4, NR4A1, KLRF1, ID3 and IL7R are, respectively, the expression levels of PLD4, NR4A1, KLRF1, ID3 and IL7R proteins or the genes encoding the proteins, measured in the biological sample isolated from the subject of interest.

In the present invention, the method of providing information may further comprise a step of substituting, into Equation 2 below, the expression levels of PLD4, ID3 and IL7R proteins or the genes encoding the proteins, measured in the biological sample isolated from the subject of interest:

[Equation 2]

$A = - a2 - b2 \times (PLD4) + c2 \times (IL7R) + d2 \times (ID3)$

wherein

a2 is a rational number of 0.5 to 0.9, preferably 0.70 to 0.85;
b2 is a rational number of 0.1 to 0.3, preferably 0.15 to 0.25;

c2 is a rational number of 0.0005 to 0.0025, preferably 0.001 to 0.002;

d2 is a rational number of 0.05 to 0.2, preferably a 0.10 to 0.15; and

PLD4, ID3 and IL7R are, respectively, the expression levels of PLD4, ID3 and IL7R proteins or the genes encoding the proteins, measured in the biological sample isolated from the subject of interest.

In the present invention, the values of PLD4, NR4A1, KLRF1, ID3 and IL7R, which are substituted into Equation 1 or 2 above, that is, the expression levels of the corresponding proteins or genes, may be normalized to the expression level of a housekeeping gene or a protein encoded by the housekeeping gene. Here, examples of the housekeeping gene include, but are not limited to, glyceraldehyde-3-phosphate desidrogenase (GAPDH), β-actin, ribosomal protein (RPL), ubiquitin (UBQ), β-tubulin, 18S ribosomal protein (18S rRNA), and phosphoglycerate kinase (PGK).

The method of providing information according to the present invention may further comprise a step of substituting the value obtained from Equation 1 or 2 above into Equation 3 below and predicting whether cancer has occurred or the likelihood of occurrence of cancer (Pr):

$$[Equation\ 3]$$

$$Pr = 1/(1+exp(-A))$$

In the present invention, when the value (Pr) obtained from Equation 3 above is greater than a rational number of 0.1 to 0.4, preferably 0.2 to 0.3, it may be predicted that cancer has occurred or is highly likely to occur.

In the method of providing information according to the present invention, predicting whether cancer has occurred or is highly likely to occur may comprise: predicting the likelihood of onset, growth, progression, or metastasis of cancer; predicting that a diseases that has occurred or is suspected of occurring in the subject of interest is cancer by distinguishing it from other diseases, in particular, pancreatic diseases (e.g., pancreatitis (including both acute and chronic), pancreatic benign tumors (lipoma or intraductal papillary mucinous neoplasm (IPMN), etc.); and predicting that cancer that has occurred or is suspected of occurring in the subject of interest is pancreatic cancer by distinguishing it from other cancer types.

In the present invention, the cancer may be thyroid cancer, parathyroid cancer, gastric cancer, ovarian cancer, colorectal cancer, pancreatic cancer, liver cancer, breast cancer, cervical cancer, lung cancer, non-small cell lung cancer, prostate cancer, gallbladder cancer, bile duct cancer, non-Hodgkin's lymphoma, Hodgkin's lymphoma, hematological cancer, bladder cancer, kidney cancer, melanoma, colon cancer, bone cancer, skin cancer, head cancer, uterine cancer, rectal cancer, brain tumor, perianal cancer, fallopian tube carcinoma, endometrial carcinoma, vaginal cancer, vulvar carcinoma, esophageal cancer, small intestine cancer, endocrine adenocarcinoma, adrenal cancer, soft tissue sarcoma, urethral cancer, penile cancer, cancer of ureter, renal cell carcinoma, renal pelvic carcinoma, central nervous system (CNS) tumor, primary CNS lymphoma, spinal cord tumor, brainstem glioma, or pituitary adenoma. Preferably, the cancer may be pancreatic cancer, but is not limited thereto and may be any type of cancer whose progression (such as tumor differentiation and/or proliferation) is dependent on the cancer cells or cancer stem cells described in the present invention.

The method of providing information according to the present invention further comprise a step of preventing, alleviating or treating cancer by administering an anticancer therapeutic drug to the subject of interest, when it is predicted that the cancer, preferably pancreatic cancer, has occurred or is highly likely to occur.

Here, the anticancer therapeutic drug may be, but is not limited to, at least one selected from the group consisting of nitrogen mustard, imatinib, oxaliplatin, rituximab, erlotinib, neratinib, lapatinib, gefitinib, vandetanib, nirotinib, semasanib, bosutinib, axitinib, masitinib, cediranib, restaurtinib, trastuzumab, gefitinib, bortezomib, sunitinib, pazopanib, toceranib, nintedanib, regorafenib, semaxanib, tivozanib, ponatinib, cabozantinib, carboplatin, sorafenib, lenvatinib, bevacizumab, cisplatin, cetuximab, viscum album, asparaginase, tretinoin, hydroxycarbamide, dasatinib, estramustine, gemtuzumab ozogamicin, ibritumomab tiuxetan, heptaplatin, methyl aminolevulinic acid, amsacrine, alemtuzumab, procarbazine, alprostadil, holmium nitrate chitosan, gemcitabine, doxifluridine, pemetrexed, tegafur, capecitabine, gimeracin, oteracil, azacitidine, methotrexate, uracil, cytarabine, 5-fluorouracil, fludarabine, enocitabine, flutamide, capecitabine, decitabine, mercaptopurine, thioguanine, cladribine, carmophor, raltitrexed, docetaxel, paclitaxel, irinotecan, belotecan, topotecan, vinorelbine, etoposide, vincristine, vinblastine, teniposide, doxorubicin, idarubicin, epirubicin, mitoxantrone, mitomycin, bleomycin, daunorubicin, dactinomycin, pirarubicin, aclarubicin, pepromycin, temsirolimus, busulfan, ifosfamide, cyclophosphamide, melphalan, altretamine, dacarbazine, thiotepa, nimustine, chlorambucil, mitolactol, leucovorin, tretonin, exemestane, amino glutesimide, anagrelide, olaparib, navelbine, fadrazole, tamoxifen, toremifene, testolactone, anastrozole, letrozole, vorozole, bicalutamide, lomustine, vorinostat, entinostat, and carmustine.

In the present invention, the anticancer therapeutic drug may be in the form of capsules, tablets, granules, injections, ointments, powders, or beverages, and the anticancer therapeutic drug may be for administration to humans.

For use, the anticancer therapeutic drug of the present invention may be formulated in the form of oral preparations such as powders, granules, capsules, tablets, and aqueous suspensions, preparations for external use, suppositories, and sterile injectable solutions, according to the respective conventional methods, without being limited thereto. The anticancer therapeutic drug of the present invention may further contain pharmaceutically acceptable carriers. As the pharmaceutically acceptable carriers, a binder, a lubricant, a disintegrant, an excipient, a solubilizer, a dispersant, a stabilizer, a suspending agent, a colorant, a flavoring agent, and the like may be used for oral administration; a buffer, a preservative, a pain-relieving agent, a solubilizer, an isotonic agent, a stabilizer, and the like may be used for injection; and a base, an excipient, a lubricant, a preservative, and the like may be used for topical administration. The pharmaceutical composition of the present invention may be prepared in various dosage forms by being mixed with the pharmaceutically acceptable carriers as described above. For example, for oral administration, the pharmaceutical composition may be formulated in the form of tablets, troches, capsules, elixirs, suspensions, syrups, wafers, or the like. For injection, the pharmaceutical composition may be formulated in the form of unit dosage ampoules or in multiple-dosage forms. In addition, the pharmaceutical composition may be formulated into solutions, suspensions, tablets, capsules, sustained-release preparations, or the like.

Meanwhile, examples of carriers, excipients and diluents suitable for formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, and mineral oil. In addition, the pharmaceutical composition of the present invention may further contain a filler, an anticoagulant, a lubricant, a wetting agent, a fragrance, an emulsifier, a preservative, or the like.

The routes of administration of the anticancer therapeutic drug according to the present invention include, but are not limited to, oral, intravenous, intramuscular, intraarterial, intramedullary, intradural, intracardiac, transdermal, subcutaneous, intraperitoneal, intranasal, gastrointestinal, topical, sublingual and intrarectal routes. Oral or parenteral administration is preferred.

In the present invention, "parenteral" includes subcutaneous, transdermal, intravenous, intramuscular, intra-articular, intra-synovial, intrasternal, intradural, intralesional and intra-cranial injection or infusion techniques. The anticancer therapeutic drug of the present invention may also be formulated as suppositories for intrarectal administration.

The dose of the anticancer therapeutic drug of the present invention may vary depending on various factors, including the patient's age, body weight, general health status, sex, and diet, the time of administration, the route of administration, excretion rate, drug combination, and the severity of a particular disease to be prevented or treated. Although the dose of the anticancer therapeutic drug may vary depending on the patient's condition and body weight, the severity of the disease, the form of drug, and the route and duration of administration, it may be appropriately selected by those skilled in the art. The anticancer therapeutic drug may be administered at a dose of 0.0001 to 50 mg/kg/day or 0.001 to 50 mg/kg/day. The anticancer therapeutic drug may be administered once a day or several times a day. The dose does not limit the scope of the present invention in any way. The anticancer therapeutic drug according to the present invention may be formulated as pills, sugar-coated tablets, capsules, liquids, gels, syrups, slurries, or suspensions.

**Advantageous Effects**

By measuring the expression level of the biomarker protein or the gene encoding the biomarker protein according to the present invention, it is possible to accurately predict or diagnose whether cancer, particularly pancreatic cancer, has occurred or is likely to occur. In addition, according to the present invention, whether a disease has occurred may be predicted or diagnosed by measuring the expression level of the biomarker from a liquid biopsy isolated from a subject, and thus it is possible to diagnose cancer simply and rapidly, but very accurately, in a non-invasive way compared to a conventional art.

**Brief Description of Drawings**

FIG. 1 shows the results of analyzing the expressions level of PLD4 in peripheral blood mononuclear cells derived from a healthy control group and a pancreatic cancer patient group in Experimental Example 2, and an ROC curve for diagnosing pancreatic cancer based on the results.

FIG. 2 shows the results of analyzing the expressions level of NR4A1 in peripheral blood mononuclear cells derived from a healthy control group and a pancreatic cancer patient group in Experimental Example 2, and an ROC curve for diagnosing pancreatic cancer based on the results.

FIG. 3 shows the results of analyzing the expressions level of KLRF1 in peripheral blood mononuclear cells derived from a healthy control group and a pancreatic cancer patient group in Experimental Example 2, and an ROC curve for diagnosing pancreatic cancer based on the results.

FIG. 4 shows the results of analyzing the expressions level of IL7R in peripheral blood mononuclear cells derived from a healthy control group and a pancreatic cancer patient group in Experimental Example 2, and an ROC curve for diagnosing pancreatic cancer based on the results.

FIG. 5 shows the results of analyzing the expression levels of ID3 in peripheral blood mononuclear cells derived from a bile duct cancer patient group and a pancreatic cancer patient group in Experimental Example 3, and an ROC curve for diagnosing pancreatic cancer based on the results.

FIG. 6 shows ROC curves obtained by analyzing the scores of efficiency of pancreatic cancer diagnosis with a combination of five markers PLD4, NR4A1, KLRF1, ID3, and IL7R for a healthy control group and a pancreatic cancer patient group in Experimental Example 4.

FIG. 7 shows ROC curves obtained by analyzing the scores of efficiency of pancreatic cancer diagnosis with a combination of five markers PLD4, NR4A1, KLRF1, ID3, and IL7R for a benign pancreatic disease group and a pancreatic cancer patient group in Experimental Example 5.

FIG. 8 shows ROC curves obtained by analyzing the scores of efficiency of pancreatic cancer diagnosis with a combination of five markers PLD4, NR4A1, KLRF1, ID3, and IL7R for a high-risk group of pancreatic disease and a pancreatic cancer patient group in Experimental Example 5.

**Best Mode**

One embodiment of the present invention is directed to a biomarker for diagnosing cancer, preferably pancreatic cancer, comprising at least one protein selected from the group consisting of PLD4 (phospholipase D family member 4), NR4A1 (nuclear receptor subfamily 4 group A member 1), KLRF1 (killer cell lectin like receptor F1), and ID3 (inhibitor of DNA binding 3, HLH protein), or a gene encoding the at least one protein.

In the present invention, the biomarker may further comprise interleukin 7 receptor (IL7R) protein or a gene encoding the same.

In the present invention, the expression level of the biomarker, etc. may be measured from a biological sample isolated from a subject of interest, preferably cells or exosomes isolated from a liquid biopsy, for example, blood, serum or plasma.

In the present invention, the diagnosing may be predicting the likelihood of occurrence, growth, progression, or metastasis of cancer, or may be distinguishing cancer from other diseases, for example, pancreatic disease, in particular, pancreatitis (including both acute and chronic), pancreatic benign tumors (lipoma or intraductal papillary mucinous neoplasm (IPMN), etc.), or may be distinguishing between cancer types, particularly, distinguishing pancreatic cancer from other cancer types.

**Mode for Invention**

Hereinafter, the present invention will be described in detail with reference to the following examples. However, the following examples are only to illustrate the present invention, and the scope of the present invention is not limited by the following examples.

**EXAMPLES**

[Experimental Example 1]

In order to identify genes with significantly increased or decreased expression levels in peripheral blood mononuclear cells (PBMCs) obtained from a total of 23 individuals (a healthy control group (n = 8) and a pancreatic cancer (PDAC) patient group (n = 15)), mRNA-seq analysis (transcriptome sequencing) was performed as described below, and among about 350 genes significantly expressed in PBMCs of the pancreatic cancer patient group compared to the control group, a total of four biomarkers in Table 1 below were discovered and validated. In an experimental method, total RNA was isolated from the sample, and then DNA contamination was removed using DNase. Next, the RNA was purified using the TruSeq Stranded mRNA LT Sample Prep kit, and then randomly fragmented for sequencing with short reads. The cleaved RNA fragments were synthesized into cDNA through a reverse transcription process, and then different adapters were ligated to both ends of the cDNA fragment. Read sequencing from both ends of the cDNA fragment was performed. The raw reads obtained through this sequencing were subjected to a preprocessing process to reduce the deviation and then mapped to the reference genome using the HISAT2 program considering splices, and then aligned reads were generated. Using the reference-based aligned reads information, transcript assembly was performed through the StringTie program. The FPKM (Fragments Per Kilobase of transcript per Million mapped reads) or RPKM (Reads Per Kilobase of transcript per Million mapped reads) values, which are normalization values considering the read count, the transcript length and the depth of coverage, were calculated using the expression level obtained by the transcript quantification of each sample, thereby extracting expression profiles. Differentially expressed genes or transcripts were selected through statistical hypothesis validation of the expression values of two or more groups under different conditions. When there is known genetic information, functional annotation and gene-set enrichment analysis based on GO and KEGG databases was performed on differentially expressed genes.

[Table 1]

| Gene_ID | Transcript_ID | Name | | Fold |
|---|---|---|---|---|
| 122618 | NM_001308174,**NM_138790** | PLD4 | phospholipase D family member 4 | - 2.007829 |
| 3164 | NM_001202233,NM_001202234,**NM_002135**,NM_173157 | NR4A1 | nuclear receptor subfamily 4 group A member 1 | - 2.859995 |
| 51348 | NM_001291822,NM_001291823,NM_001 366534,**NM_016523** | KLRF1 | killer cell lectin like receptor F1 | - 2.460315 |
| 3399 | **NM_002167** | ID3 | inhibitor of DNA binding 3, HLH protein | 2.522447 |

As shown in Table 1, it could be confirmed that the mRNA expression levels of PLD4, NR4A1 and KLRF1 decreased in the pancreatic cancer patients compared to the healthy control group, whereas the mRNA expression level of ID3 increased.

[Experimental Example 2]

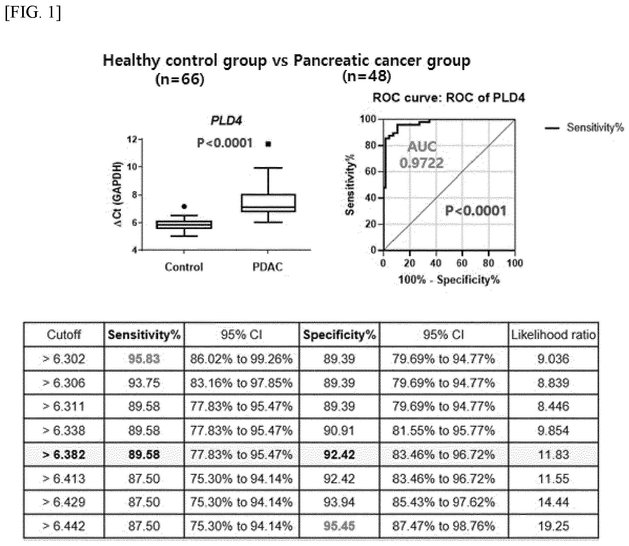

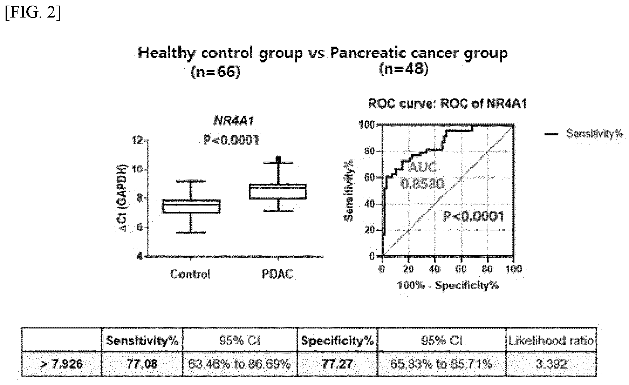

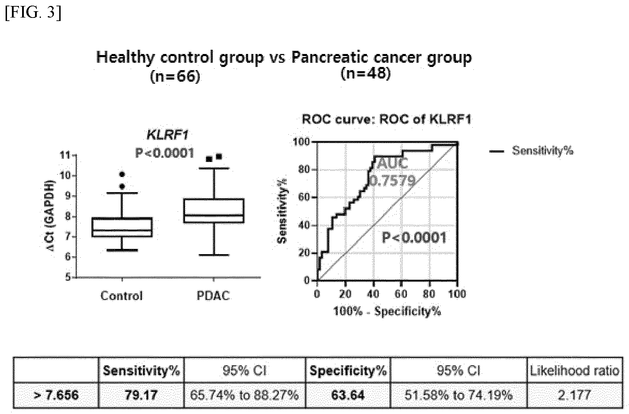

Peripheral blood mononuclear cells (PBMCs) were isolated from the blood of each of a healthy control group and a pancreatic cancer (PDAC) patient group, and then RNA was isolated therefrom (Qiagen, USA). Next, cDNA was synthesized from the RNA using a PrimeScript RT Master Mix (Perfect Real Time, Takara #RR036A). The mRNA expression levels of PLD4, NR4A1, KLRF1, and IL7R in each group were measured by performing PCR using a StepOnePlus PCR system (Applied Biosystems), and the results are shown in FIGS. 1 to 4. Primer sequences used in the PCR are shown in Table 2 below. In the following analysis, sensitivity indicates the probability that a person with a disease will show a positive outcome in diagnosis, specificity indicates the probability that a person without a disease will show a negative outcome in diagnosis, and AUC (Area Under the ROC Curve) is a measure for evaluating the performance of a predictor.

[Table 2]

| Gene | Form | Sequence (5' -> 3') |
|---|---|---|
| PLD4 (Ref. NM_138790) | Forward primer | CAA ATT CTG GGT TGT GGA TGG |
| | Reverse primer | AGG TGG CTG CAG TTA TAG ATG |
| NR4A1 (Ref. NM_002135.5) | Forward primer | GACAACGCTTCATGCCAG |
| | Reverse primer | TTGTTAGCCAGGCAGATGTAC |
| KLRF1 (Ref. NM_016523) | Forward primer | GAA AAG GAG TTC TGC CCA AAC |
| | Reverse primer | AGA AAC CAA CAG GAT CAA GGA G |
| IL7R (Ref. NM_002185.5) | Forward primer | GTA GTC ATC ACT CCA GAA AGC |
| | Reverse primer | ACC TGG AAG AGG AGA GAA TAG |

As shown in FIGS. 1 to 4, it could be confirmed that the mRNA expression levels of PLD4, NR4A1 and KLRF1 significantly decreased (ΔCt increased) in the peripheral blood mononuclear cells (PBMCs) derived from the pancreatic cancer patient group compared to the healthy control group, the mRNA expression level of IL7R significantly increased (ΔCt decreased) in the peripheral blood mononuclear cells (PBMCs). In addition, it could be seen through the ROC curve that the accuracy of pancreatic cancer diagnosis was excellent when these biomarkers were used.

[Experimental Example 3]

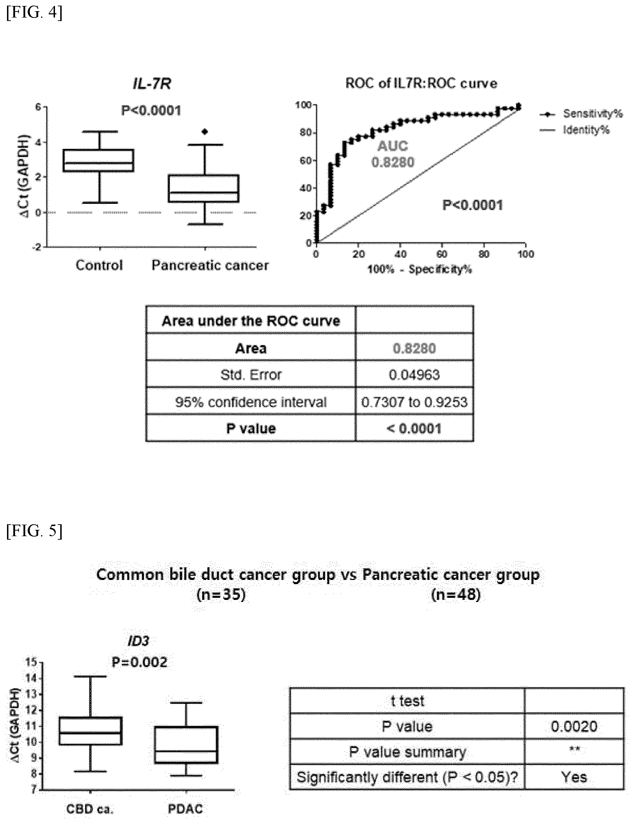

Peripheral blood mononuclear cells (PBMCs) were isolated from the blood of each of a common bile duct (CBD) cancer patient group and a pancreatic cancer (PDAC) patient group, and then RNA was isolated therefrom (Qiagen, USA). Next, cDNA was synthesized from the RNA using a PrimeScript RT Master Mix (Perfect Real Time, Takara #RR036A). The mRNA expression level of ID3 in each group was measured by performing PCR using a StepOnePlus PCR system (Applied Biosystems), and the results are shown in FIG. 5. Primer sequences used in the PCR are shown in Table 3 below.

[Table 3]

| Gene | Form | Sequence (5' -> 3') |
|---|---|---|
| ID3 (Ref. NM_002167) | Forward primer | GAC TAC ATT CTC GAC CTG CAG |
| | Reverse primer | TCG TTG GAG ATG ACA AGT TCC |

As shown in FIG. 5, it could be confirmed that the mRNA expression level of ID3 significantly increased ($\Delta$Ct decreased) in the peripheral blood mononuclear cells (PBMCs) derived from the pancreatic cancer patient group compared to the bile duct cancer patient group.

[Experimental Example 4]

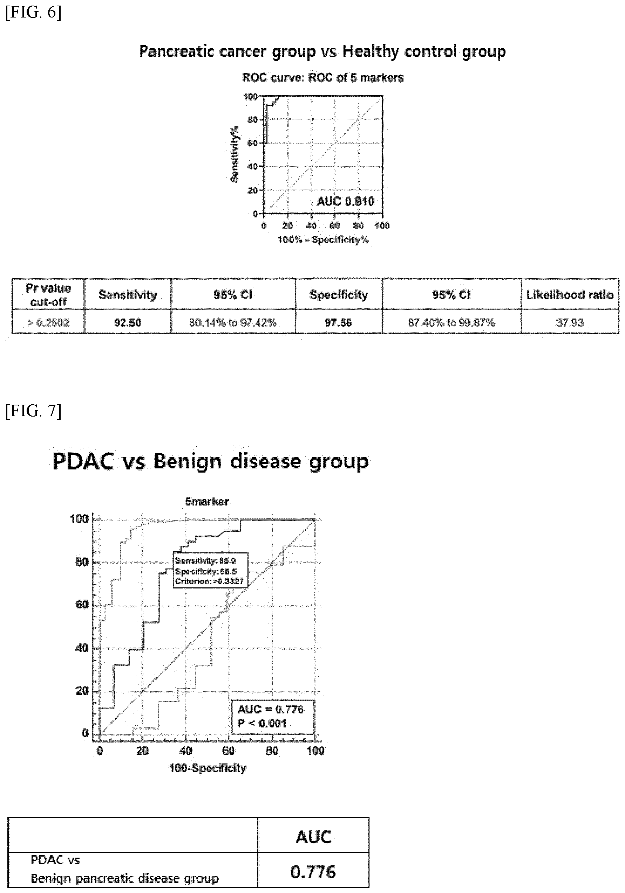

Based on the results of Experimental Examples 2 and 3, the scores of efficiency of diagnosis with a combination of five markers PLD4, NR4A1, KLRF1, ID3, and IL7R to predict the healthy control group and the pancreatic cancer patient group were analyzed. SASSAS (version 9.3, SAS Inc., Cary, NC, USA) was used as the analysis software, and the following Equations 3 and 4 were derived by analyzing the predicted probability (Pr), and the ROC curve obtained by the analysis is shown in FIG 6.

[Equation 3]

$$Pr = 1/(1+\exp(-A))$$

[Equation 4]

$$A=1.95710-0.21170X(PLD4)-0.024072X(NR4A1)-$$

$$0.012005X(KLRF1)+0.00097174X(IL7R)+0.086073X(ID3)$$

In Equation 4 above, PLD4, NR4A1, KLRF1, ID3, and IL7R are the mRNA expression levels ($\Delta$Ct values) of PLD4, NR4A1, KLRF1, ID3, and IL7R measured in the peripheral blood mononuclear cells of each group, respectively.
As a result, as shown in FIG. 6, as a result of analyzing the predicted probability (Pr) with a combination of five markers PLD4, NR4A1, KLRF1, ID3, and IL7R for prediction of the healthy control group and the pancreatic cancer patient group, it could be seen that the diagnostic accuracy (AUC) was 0.910, the sensitivity was 92.50%, and the specificity was 97.56%, which were all very high values, suggesting that, when the combination of five markers PLD4, NR4A1, KLRF1, ID3, and IL7R is used, whether pancreatic cancer has occurred or the likelihood of occurrence of pancreatic cancer can be predicted with excellent accuracy.

[Experimental Example 5]

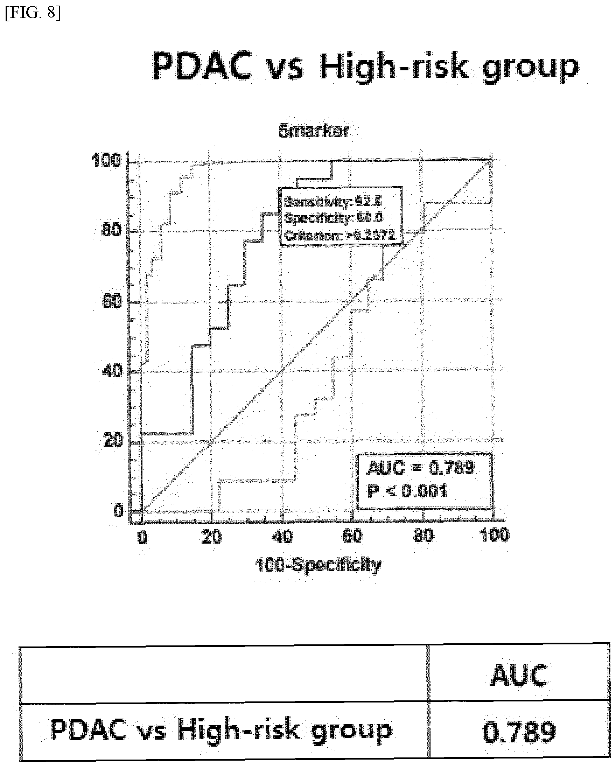

Based on the results of Experimental Examples 2 and 3, the scores of efficiency of diagnosis with the combination of five markers PLD4, NR4A1, KLRF1, ID3 and IL7R were analyzed for diagnosis of the pancreatic cancer patient group versus a benign pancreatic disease group (acute pancreatitis and pancreatic lipoma) or a high-risk group of pancreatic disease (chronic pancreatitis and IPMN). SASSAS (version 9.3, SAS Inc., Cary, NC, USA) was used as the analysis software, and ROC curves obtained by the analysis are shown in FIGS. 7 and 8.
As shown in FIGS. 7 and 8, it was confirmed that the accuracy (AUC) of diagnosis of the pancreatic cancer patient group versus the benign pancreatic disease group (acute pancreatitis and pancreatic lipoma) with the combination of the five markers PLD4, NR4A1, KLRF1, ID3, and IL7R was 0.776 and the accuracy (AUC) of diagnosis of the pancreatic cancer patient group versus the high-risk group of pancreatic disease (chronic pancreatitis and IPMN) with the combination of the five markers was 0.789, suggesting that the combination of the five markers PLD4, NR4A1, KLRF1, ID3, and IL7R has significance as a biomarker for diagnosing pancreatic cancer by distinguishing it from other pancreatic diseases.

[Experimental Example 6]

Peripheral blood mononuclear cells (PBMC) were isolated from a total of 272 individuals (a pancreatic cancer (PDAC) patient group (n = 50) and a non-pancreatic cancer patient group (n = 222)) shown in Table 4 below, and then the expression levels of PLD4, ID3 and IL7R were measured using the primers for PLD4, ID3 and IL7R shown in Tables 2 and 3 above and the probes shown in Table 5 below. Next, in the same manner as in Experimental Examples 4 and 5, logistic regression models for PLD4, ID3, and IL7R markers alone or in combination were built as predictive models for distinguishing the pancreatic cancer patient group from the non-pancreatic cancer patient group. At this time, Equations 3 and 5 below were derived through predicted probability (Pr) analysis. The results of the analysis are shown in Tables 6 and 7 below. In Table 7 below, to determine the predictive power (area under the curve (AUC)) and diagnostic power (sensitivity, specificity, accuracy, PPV, NPV) of each model, the cut-off value was set as the point at which Youden's index (=sensitivity + specificity - 1) was maximized.

[Table 4]

| Patient groups | |
| --- | --- |
| Pancreatic cancer patient group (n=50) | Non-pancreatic cancer patient group (n=222)<br>- Normal persons (n=61)<br>- High-risk group (chronic pancreatitis, pancreatic cyst, pancreatic duct dilatation, etc.) (n=56)<br>- Benign pancreatic disease group (n=48)<br>- Other digestive system cancer groups (bile duct cancer, gastric cancer, colorectal cancer, liver cancer, etc.) (n=54)<br>- Others (n=3) |

[Equation 3]

$$Pr = 1/(1+exp(-A))$$

[Equation 5]

$$A = -0.789445 - 0.229438 \times (PLD4) + 0.001251 \times (IL7R) + 0.134571 \times (ID3)$$

In Equation 5, PLD4, ID3, and IL7R are, respectively, the ratios of the copy numbers of the PLD4, ID3 and IL7R markers to the GAPDH copy number (marker copy numbers/GAPDH copy number), measured for the peripheral blood mononuclear cells from each group.

[Table 5]

| | Name | Sequence (5'-3') | Reporter 5' | Quencher 3' |
| --- | --- | --- | --- | --- |
| 1 | PLD4-FAM | TCT GAC GCA GGT GAA GGA GCT TG | FAM | ZEN/IBFQ |
| 2 | IL7R-FAM | CAG TTG GAA GTG AAT GGA TCG CAG C | FAM | ZEN/IBFQ |
| 3 | ID3-FAM | CTC GGC TGT CTG GAT GGG AAG G | FAM | ZEN/IBFQ |

[Table 6]

| Variables | Single marker model | | | | | PLD4+ID3+IL7R combination marker model | | | |
|---|---|---|---|---|---|---|---|---|---|
| | Intercept | p-value | Beta (SE) | OR (95% CI) | p-value | Beta (SE) | OR (95% CI) | p-value | VIF |
| Intercept | - 0.034331 (0.3 140) | 0.91 29 | - 0.096451 (0.0 208) | 0.908(0.8 72-0.946) | <.00 01 | - 0.229438 (0.0 395) | 0.795(0.7 36-0.859) | <.00 01 | 1.15 54 |
| PLD4 | - 2.165325 (0.3 397) | <.00 01 | 0.000603 (0.0 003) | 1.001(1.0 00-1.001) | 0.01 79 | 0.001251 (0.0 004) | 1.001(1.0 00-1.002) | 0.00 25 | 1.68 04 |
| IL7R | - 2.116365 (0.2 716) | <.00 01 | 0.072584 (0.0 237) | 1.075(1.0 26-1.126) | 0.00 22 | 0.134571 (0.0 392) | 1.144(1.0 59-1.235) | 0.00 06 | 1.68 35 |
| ID3 | - 0.034331 (0.3 140) | 0.91 29 | - 0.096451 (0.0 208) | 0.908(0.8 72-0.946) | <.00 01 | - 0.229438 (0.0 395) | 0.795(0.7 36-0.859) | <.00 01 | 1.15 54 |

[Table 7]

| Model | Optimal cut-off point | AUC (95% CI) | Sensitivity(95 % CI) | Specificity(9 5% CI) | Accuracy (95% CI) | PPV (95% CI) | NPV (95% CI) |
|---|---|---|---|---|---|---|---|
| PLD4 | <18.8693 | 0.727(0.66 2-0.793) | 0.900 (0.817-0.983) | 0.523 (0.457-0.588) | 0.592 (0.53 4-0.650) | 0.298(0.22 5-0.371) | 0.959(0.92 3-0.994) |
| IL7R | >1025.40 733 | 0.621(0.53 3-0.709) | 0.660 (0.529-0.791) | 0.568 (0.502-0.633) | 0.585 (0.52 6-0.643) | 0.256(0.18 1-0.331) | 0.881(0.82 8-0.934) |
| ID3 | >8.7021 | 0.640(0.54 3-0.737) | 0.620 (0.485-0.755) | 0.716 (0.657-0.776) | 0.699 (0.64 4-0.753) | 0.330(0.23 5-0.425) | 0.893(0.84 8-0.939) |
| PLD4+ID3 +IL7R | >0.22016 | 0.860(0.81 4-0.907) | 0.840 (0.738-0.942) | 0.788 (0.735-0.842) | 0.798 (0.75 0-0.846) | 0.472(0.36 8-0.576) | 0.956(0.92 7-0.986) |

As a result, as shown in Tables 6 and 7 above, it was confirmed that the p-values of the regression coefficients of all the markers in the PLD4, IL7R, and ID3 single marker models in distinguishing pancreatic cancer from non-pancreatic cancer were less than 0.05, which were statistically significant, and that the p-values of the regression coefficients of the three markers in the multinomial logistic regression model of PLD4, IL7R, and ID3 built accordingly were also less than 0.05, which were statistically significant. In addition, it could be confirmed that the diagnostic accuracy (AUC), sensitivity, and specificity values were all high, and that these values became higher when the three markers were combined together.

This suggests that, when the single markers PLD4, IL7R, and ID3 and a combination thereof are used, it is possible to diagnose pancreatic cancer by distinguishing it from other diseases.

Although the present invention has been described in detail with reference to the specific features, it will be apparent to those skilled in the art that this description is only of a preferred embodiment thereof, and does not limit the scope of the present invention. Thus, the substantial scope of the present invention will be defined by the appended claims and equivalents thereto.

**Industrial Applicability**

The present invention relates to a composition and a kit for diagnosing pancreatic cancer, and a method of providing information for diagnosis of pancreatic cancer.

**Claims**

1. A composition for diagnosing pancreatic cancer comprising, as an active ingredient, an agent for measuring an expression level of at least one protein selected from the group consisting of PLD4 (phospholipase D family member 4), NR4A1 (nuclear receptor subfamily 4 group A member 1), KLRF1 (killer cell lectin like receptor F1), and ID3 (inhibitor of DNA binding 3, HLH protein), or a gene encoding the at least one protein.

2. The composition according to claim 1, further comprising an agent for measuring an expression level of IL7R (interleukin 7 receptor) protein or a gene encoding the IL7R protein.

3. The composition according to claim 1, wherein the agent for measuring the expression level of the protein comprises at least one selected from the group consisting of an antibody, an oligopeptide, a ligand, a peptide nucleic acid (PNA), and an aptamer, which bind specifically to the protein.

4. The composition according to claim 1, wherein the agent for measuring the expression level of the gene comprises at least one selected from the group consisting of a primer, a probe, and an antisense nucleotide, which bind specifically to the gene.

5. The composition according to claim 1, wherein the composition is for a liquid biopsy isolated from a subject of interest.

6. The composition according to claim 1, wherein the composition is for predicting a likelihood of occurrence, growth, progression or metastasis of cancer, or for distinguishing between cancer and pancreatic disease, or for distinguishing between cancer types.

7. A kit for diagnosing pancreatic cancer comprising the composition according to any one of claims 1 to 6.

8. The kit according to claim 7, wherein the kit is an RT-PCR kit, a DNA chip kit, an ELISA kit, a protein chip kit, a rapid kit, or a multiple reaction monitoring (MRM) kit.

9. A method of providing information for diagnosis of pancreatic cancer, the method comprising a step of measuring an expression level of at least one protein selected from the group consisting of PLD4 (phospholipase D family member 4), NR4A1 (nuclear receptor subfamily 4 group A member 1), KLRF1 (killer cell lectin like receptor F1), and ID3 (inhibitor of DNA binding 3, HLH protein), or a gene encoding the at least one protein, in a biological sample isolated from a subject of interest.

10. The method according to claim 9, wherein the biological sample is at least one selected from the group consisting of whole blood, leukocytes, peripheral blood mononuclear cells, buffy coat, plasma, serum, sputum, tears, mucus, nasal washes, nasal aspirate, breath, urine, semen, saliva, peritoneal washings, ascites, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extracts, and cerebrospinal fluid.

11. The method according to claim 9, wherein the biological sample is a liquid biopsy.

12. The method according to claim 9, further comprising a step of measuring an expression level of IL7R protein or a gene encoding the IL7R protein in the biological sample isolated from the subject of interest.

13. The method according to claim 9, wherein the agent for measuring the expression level of the protein comprises at least one selected from the group consisting of an antibody, an oligopeptide, a ligand, a peptide nucleic acid (PNA), and an aptamer, which bind specifically to the protein.

14. The method according to claim 9, wherein the measuring the expression level of the protein is performed by protein chip assay, immunoassay, ligand binding assay, MALDI-TOF (Matrix Assisted Laser Desorption/Ionization Time of Flight) mass spectrometry, SELDI-TOF (Surface Enhanced Laser Desorption/Ionization Time of Flight) mass spectrometry, radioimmunoassay, radial immunodiffusion, Ouchterlony immunodiffusion, Rocket immunoelectrophoresis, immunohistostaining, complement fixation test, 2-D electrophoresis, liquid chromatography-mass spectrometry (LC-MS), liquid chromatography-mass spectrometry/mass spectrometry (LC-MS/MS), Western blotting, enzyme-linked immunosorbent assay (ELISA), or a multiple reaction monitoring (MRM) method.

**15.** The method according to claim 9, wherein the agent for measuring the expression level of the gene comprises at least one selected from the group consisting of a primer, a probe, and an antisense nucleotide, which bind specifically to the gene.

**16.** The method according to claim 9, wherein the measuring the expression level of the gene is performed by reverse transcription polymerase reaction (RT-PCR), competitive RT-PCR, real-time RT-PCR, RNase protection assay (RPA), Northern blotting, or DNA chip assay.

**17.** The method according to claim 9, comprising predicting that pancreatic cancer has occurred or is highly likely to occur in the subject of interest, when the expression level of at least one protein selected from the group consisting of PLD4, NR4A1, and KLRF1, or the gene encoding the at least one protein, measured in the biological sample isolated from the subject of interest, is lower than a control.

**18.** The method according to claim 12, comprising predicting that pancreatic cancer has occurred or is highly likely to occur in the subject of interest, when the expression level of at least one protein selected from among ID3 and IL7R, or the gene encoding the protein, measured in the biological sample isolated from the subject of interest, is higher than a control.

**19.** The method according to claim 17 or 18, wherein the predicting that pancreatic cancer has occurred or is highly likely to occur comprises predicting a likelihood of occurrence, growth, progression or metastasis of pancreatic cancer, or distinguishing pancreatic cancer from pancreatic disease, or distinguishing pancreatic cancer from other cancer types.

**20.** A biomarker for diagnosing pancreatic cancer comprising at least one protein selected from the group consisting of PLD4 (phospholipase D family member 4), NR4A1 (nuclear receptor subfamily 4 group A member 1), KLRF1 (killer cell lectin like receptor F1), and ID3 (inhibitor of DNA binding 3, HLH protein), or a gene encoding the at least one protein.

**21.** The biomarker according to claim 20, further comprising IL7R (interleukin 7 receptor) protein or a gene encoding the IL7R protein.

[FIG. 1]

Healthy control group vs Pancreatic cancer group
(n=66) (n=48)

| Cutoff | Sensitivity% | 95% CI | Specificity% | 95% CI | Likelihood ratio |
|--------|--------------|--------|--------------|--------|------------------|
| > 6.302 | 95.83 | 86.02% to 99.26% | 89.39 | 79.69% to 94.77% | 9.036 |
| > 6.306 | 93.75 | 83.16% to 97.85% | 89.39 | 79.69% to 94.77% | 8.839 |
| > 6.311 | 89.58 | 77.83% to 95.47% | 89.39 | 79.69% to 94.77% | 8.446 |
| > 6.338 | 89.58 | 77.83% to 95.47% | 90.91 | 81.55% to 95.77% | 9.854 |
| > 6.382 | 89.58 | 77.83% to 95.47% | 92.42 | 83.46% to 96.72% | 11.83 |
| > 6.413 | 87.50 | 75.30% to 94.14% | 92.42 | 83.46% to 96.72% | 11.55 |
| > 6.429 | 87.50 | 75.30% to 94.14% | 93.94 | 85.43% to 97.62% | 14.44 |
| > 6.442 | 87.50 | 75.30% to 94.14% | 95.45 | 87.47% to 98.76% | 19.25 |

[FIG. 2]

Healthy control group vs Pancreatic cancer group
(n=66)                                    (n=48)

| | Sensitivity% | 95% CI | Specificity% | 95% CI | Likelihood ratio |
|---|---|---|---|---|---|
| > 7.926 | 77.08 | 63.46% to 86.69% | 77.27 | 65.83% to 85.71% | 3.392 |

[FIG. 3]

Healthy control group vs Pancreatic cancer group
(n=66)                                  (n=48)

| | Sensitivity% | 95% CI | Specificity% | 95% CI | Likelihood ratio |
|---|---|---|---|---|---|
| > 7.656 | 79.17 | 65.74% to 88.27% | 63.64 | 51.58% to 74.19% | 2.177 |

[FIG. 4]

| Area under the ROC curve | |
|---|---|
| **Area** | 0.8280 |
| Std. Error | 0.04963 |
| 95% confidence interval | 0.7307 to 0.9253 |
| **P value** | **< 0.0001** |

[FIG. 5]

**Common bile duct cancer group vs Pancreatic cancer group**
(n=35)                          (n=48)

| t test | |
|---|---|
| P value | 0.0020 |
| P value summary | ** |
| Significantly different (P < 0.05)? | Yes |

[FIG. 6]

**Pancreatic cancer group vs Healthy control group**

| Pr value cut-off | Sensitivity | 95% CI | Specificity | 95% CI | Likelihood ratio |
|---|---|---|---|---|---|
| > 0.2602 | 92.50 | 80.14% to 97.42% | 97.56 | 87.40% to 99.87% | 37.93 |

[FIG. 7]

**PDAC vs Benign disease group**

| | AUC |
|---|---|
| PDAC vs Benign pancreatic disease group | 0.776 |

[FIG. 8]

## PDAC vs High-risk group

5marker

Sensitivity: 92.5
Specificity: 60.0
Criterion: >0.2372

AUC = 0.789
P < 0.001

100-Specificity

| | AUC |
|---|---|
| PDAC vs High-risk group | 0.789 |

## INTERNATIONAL SEARCH REPORT

| | International application No. |
|---|---|
| | **PCT/KR2022/003242** |

**A. CLASSIFICATION OF SUBJECT MATTER**

**C12Q 1/6886**(2018.01)i; **G01N 33/574**(2006.01)i

According to International Patent Classification (IPC) or to both national classification and IPC

**B. FIELDS SEARCHED**

Minimum documentation searched (classification system followed by classification symbols)

C12Q 1/6886(2018.01); C40B 30/04(2006.01); C40B 30/06(2006.01); G01N 33/574(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: PLD4(phospholipase D family member 4), NR4A1(nuclear receptor subfamily 4 group a member 1), KLRF1(killer cell lectin like receptor f1), ID3(inhibitor of dna binding 3), IL7R(initerleukin 7 receptor), 췌장암(pancreatic cancer), 암(cancer), 종양(tumor), 진단(diagnosis), 키트(kit), 바이오마커(biomarker)

**C. DOCUMENTS CONSIDERED TO BE RELEVANT**

| Category* | Citation of document, with indication, where appropriate, of the relevant passages | Relevant to claim No. |
|---|---|---|
| X | WO 2018-205035 A1 (GENOMEDX BIOSCIENCES, INC) 15 November 2018 (2018-11-15)<br>See abstract, claims 1, 15-18, 20-21, 23-26, 30, 35, 43 and 47-50, paragraphs [0003], [0007]-[0009], [0011]-[0012], [0014], [0076] and [0201], and table 1. | 1,3-11,13-17,19-20 |
| Y | | 2,12,18,21 |
| Y | KR 10-2020-0134071 A (ACURASYSBIO CO., LTD.) 01 December 2020 (2020-12-01)<br>See abstract, claims 1, 3, 11 and 16, and table 1. | 2,12,18,21 |
| A | 이승옥. 핵수용체 NR4A1을 표적하는 췌장암 치료용 천연 식의약 소재 발굴. 정부과제 최종보고서. 2017 (LEE, Syng-Ook. Natural anticancer nutraceuticals targeting the nuclear receptor NR4A1 in human pancreatic cancer. Government Project Final Report.) See pages 1-15.<br>See entire document. | 1-21 |
| A | US 2002-0182614 A1 (GILLIS, K. A. et al.) 05 December 2002 (2002-12-05)<br>See entire document. | 1-21 |

☑ Further documents are listed in the continuation of Box C.   ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **15 June 2022** | **21 June 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.

**PCT/KR2022/003242**

**C.    DOCUMENTS CONSIDERED TO BE RELEVANT**

| Category* | Citation of document, with indication, where appropriate, of the relevant passages | Relevant to claim No. |
|-----------|-----------------------------------------------------------------------------------|-----------------------|
| A | EP 3336547 A1 (LIONEX GMBH) 20 June 2018 (2018-06-20)<br>See entire document. | 1-21 |

Form PCT/ISA/210 (second sheet) (July 2019)

**INTERNATIONAL SEARCH REPORT**

International application No.

**PCT/KR2022/003242**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a.  ☑  forming part of the international application as filed:

        ☑  in the form of an Annex C/ST.25 text file.

        ☐  on paper or in the form of an image file.

    b.  ☐  furnished together with the international application under PCT Rule 13*ter*.1(a) for the purposes of international search only in the form of an Annex C/ST.25 text file.

    c.  ☐  furnished subsequent to the international filing date for the purposes of international search only:

        ☐  in the form of an Annex C/ST.25 text file (Rule 13*ter*.1(a)).

        ☐  on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (July 2019)

**INTERNATIONAL SEARCH REPORT**
**Information on patent family members**

International application No.

**PCT/KR2022/003242**

| Patent document cited in search report | | | Publication date (day/month/year) | Patent family member(s) | | | Publication date (day/month/year) |
|---|---|---|---|---|---|---|---|
| WO | 2018-205035 | A1 | 15 November 2018 | EP | 3622087 | A1 | 18 March 2020 |
| | | | | US | 1078542 | B2 | 03 August 2021 |
| | | | | US | 2019-0017123 | A1 | 17 January 2019 |
| KR | 10-2020-0134071 | A | 01 December 2020 | EP | 3974541 | A1 | 30 March 2022 |
| | | | | KR | 10-2022-0045945 | A | 13 April 2022 |
| | | | | KR | 10-2384933 | B1 | 11 April 2022 |
| | | | | WO | 2020-235943 | A1 | 26 November 2020 |
| US | 2002-0182614 | A1 | 05 December 2002 | EP | 1356107 | A2 | 29 October 2003 |
| | | | | JP | 2004-526424 | A | 02 September 2004 |
| | | | | WO | 02-44417 | A2 | 06 June 2002 |
| | | | | WO | 02-44417 | A3 | 24 July 2003 |
| EP | 3336547 | A1 | 20 June 2018 | None | | | |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 100819122

- KR 20120082372

### Non-patent literature cited in the description

- **KOHLER ; MILSTEIN.** *European Journal of Immunology,* 1976, vol. 6, 511-519
- **CLACKSON et al.** *Nature,* 1991, vol. 352, 624-628
- **MARKS et al.** *J. Mol. Biol.,* 1991, vol. 222 (58), 1-597
- **NIELSEN P E ; EGHOLM M ; BERG R H ; BUCHARDT O.** Sequence-selective recognition of DNA by strand displacement with a thymine-substituted polyamide. *Science,* December 1991, vol. 254 (5037), 1497-1500

- **BOCK L C et al.** *Nature,* 1992, vol. 355 (6360), 5646
- **HOPPE-SEYLER F ; BUTZ K.** Peptide aptamers: powerful new tools for molecular medicine. *J Mol Med.,* 2000, vol. 78 (8), 42630
- **COHEN B A ; COLAS P ; BRENT R.** An artificial cell-cycle inhibitor isolated from a combinatorial library. *Proc Natl Acad Sci USA.,* 1998, vol. 95 (24), 142727
- **J WEILER ; J HUNZIKER.** *J Hall Gene Therapy,* 2006, vol. 13, 496-502